(19) Europäisches Patentamt
European Patent Office
Office européen des brevets

(11) **EP 3 056 146 A1**

(12) **EUROPÄISCHE PATENTANMELDUNG**

(43) Veröffentlichungstag:
**17.08.2016 Patentblatt 2016/33**

(21) Anmeldenummer: **16161365.8**

(22) Anmeldetag: **24.05.2013**

(51) Int Cl.:
*A61B 5/145* (2006.01)    *A61B 5/1455* (2006.01)
*A61B 5/00* (2006.01)    *G01J 5/02* (2006.01)
*B29C 51/08* (2006.01)    *G01K 1/08* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.06.2012 AT 6622012**

(62) Dokumentnummer(n) der früheren Anmeldung(en) nach Art. 76 EPÜ:
**13731024.9 / 2 858 568**

(71) Anmelder: **VASEMA DIAGNOSTICS AG**
**8050 Zürich (CH)**

(72) Erfinder: **Hagl, Peter**
**8050 Zurich (CH)**

(74) Vertreter: **Wildhack & Jellinek**
**Patentanwälte**
**Landstraßer Hauptstraße 50**
**1030 Wien (AT)**

Bemerkungen:
Diese Anmeldung ist am 21.03.2016 als Teilanmeldung zu der unter INID-Code 62 erwähnten Anmeldung eingereicht worden.

(54) **ABDECKKAPPE**

(57) Die Erfindung betrifft eine Abdeckkappe (10), insbesondere zum Aufsetzen auf ein Hautanalysegerät, umfassend eine von einer Umfangskante (4) begrenzte Stirnwand (1) sowie eine an der Umfangskante (4) der Stirnwand (1) anschließende Mantelwand (2), wobei zumindest ein Teil der Abdeckkappe (10) eine Dicke ($d_2$) aufweist, die geringer ist als diejenige Dicke ($d_3$) der Mantelwand (2) in einem Abschnitt (9) der Mantelwand (2), der im von der Stirnwand (1) abgewandten Bereich oder Ende der Mantelwand (2) liegt, wobei die Stirnwand (1) gasdurchlässig ausgebildet ist.

Fig. 1a

Fig. 1b

**Beschreibung**

Die Erfindung betrifft eine Abdeckkappe, insbesondere zum Aufsetzen auf ein Hautanalysegerät gemäß dem Oberbegriff des Patentanspruches 1. Weiters betrifft die Erfindung ein Messgerät, insbesondere zur Hautanalyse gemäß Patentanspruch 15. Ferner betrifft die Erfindung ein Verfahren zur Herstellung einer Abdeckkappe gemäß dem Oberbegriff des Patentanspruches 23.

Hintergrund der Erfindung ist die Verwendung von Abdeckkappen, auch Hygienekappen genannt, zur Abdeckung von Sensoren eines Messgeräts. Hierbei sind Abdeckkappen unerlässlich, um steril oder unsteril, sauber Menschen, Tiere oder Materialien zu vermessen, ohne den mit der Abdeckkappe abgedeckten Sensor oder dass von der Abdeckkappe abgedeckte Messgerät zu verschmutzen. Solche Abdeckkappen werden im Stand der Technik, beispielsweise bei einem Infrarotthermometer verwendet, wobei der Infrarotsensor zum Schutz vor Verschmutzungen mit einer Abdeckkappe abgedeckt ist. Die Verwendung von Abdeckkappen dient dazu, Krankheiten nicht auf andere Menschen oder Körperteile zu übertragen.

Die aus dem Stand der Technik verfügbaren Abdeckkappen weisen jedoch in vielerlei Hinsicht Nachteile auf. Einerseits besteht das Problem, dass bekannte Abdeckkappen viel Raum benötigen und schlecht faltbar sind. Ferner besteht auch das Problem, mit Abdeckkappen hygienisch zu hantieren, sowie eine optimale Passform der Hygienekappe zu erzielen, insbesondere das Spannen der Abdeckkappe, um den sensitiven Teil eines Messgeräts ermöglichen. Weiters besteht das Problem, eine Abdeckkappe zu entsorgen, ohne diese zu berühren.

Aus dem Stand der Technik ist es bekannt, dass Hygienekappen auf einer möglichst gleichmäßigen und gleichdicken Material, vorzugsweise Polyethylen oder Polypropylen, hergestellt werden, wobei derjenige Oberflächenbereich der jeweiligen Abdeckkappe mittels eines Mikrotonmeters oder mittels Laser im Bereich der Stirnwand verdünnt wird, durch die der die jeweilige Temperatur charakterisierende Infrarotstrahl hindurch dringt.

Weiters kann die Verdünnung der Oberfläche im Bereich der Stirnwand auch mittels Extrudieren einer Schmelze mit anschließenden Einsetzen einer Folie verwirklicht werden. Die so erzielten Abdeckkappen sind sehr steif, was einerseits das Spannen der Abdeckkappen um das Messgerät erschwert und auch ein platzsparendes Recycling durch Zusammenfalten und Zusammendrücken auf einen flachen Gegenstand unmöglich macht.

Somit besteht die Aufgabe der Erfindung darin, eine Abdeckkappe zu schaffen, die das Messgerät vorteilhaft abdeckt und sich der Form des Messgeräts optimal anpasst und die einfach und mit geringem Volumen zu entsorgen ist. Die Erfindung löst diese Aufgabe bei einer Abdeckkappe der eingangs genannten Art mit dem kennzeichnenden Merkmal des Patentanspruchs 1.

Eine weitere Aufgabe der Erfindung liegt in der Bereitstellung eines vorteilhaften Verfahrens zur Herstellung einer Abdeckkappe. Die Erfindung löst diese Aufgabe beim Verfahren der eingangs genannten Art mit dem kennzeichnenden Merkmal des Patentanspruchs 23.

Weiters ist es Aufgabe der Erfindung ein Messgerät zu schaffen, das eine vorteilhafte und berührungsfreie Entsorgung mit einer erfindungsgemäßen Abdeckkappe ermöglicht. Die Erfindung löst diese Aufgabe bei einem Messgerät der eingangs genannten Art mit dem kennzeichnenden Merkmal des Patentanspruchs 15.

Erfindungsgemäß ist bei einer Abdeckkappe, insbesondere zum Aufsetzen auf ein Hautanalysegerät, umfassend eine von einer Umfangskante begrenzte Stirnwand sowie eine an der Umfangskante der Stirnwand anschließende Mantelwand, vorgesehen, dass die Stirnwand gasdurchlässig ausgebildet ist.

Eine besondere Weiterbildung der Erfindung sieht vor, dass die Mantelwand zumindest einen umlaufenden Abschnitt aufweist, dessen Dicke geringer ist als die Dicke der Mantelwand im Abschnitt des von der Stirnwand abgewandten Endes der Mantelwand. Durch diese Maßnahme ist im Bereich des umlaufenden Abschnitts eine gewisse Flexibilität gegeben, mit der eine optimale Anpassung der Abdeckkappe an das jeweilige Messgerät ermöglicht wird.

Ganz grundsätzlich ist es bei jeder Art von Abdeckkappe, insbesondere bei sämtlichen vorstehend genannten Weiterbildungen der Erfindung, von Vorteil, wenn dass die Stirnwand an zumindest einer Stelle eine Dicke im Bereich von weniger als 100 $\mu$m, vorzugsweise zwischen 0,1 $\mu$m und 20 $\mu$m, insbesondere zwischen 5 $\mu$m und 10 $\mu$m, aufweist. Hierdurch ist es möglich, eine Stirnwand zu erstellen, durch die Gase hindurch diffundieren können und eine Abdeckkappe auch zum Schutz des Sensors vor schädlichen Stoffen möglich ist, ohne die gesamte Funktion des Messgeräts zu beeinträchtigen, da durch die Ausbildung Stirnwand die zu messenden Stoffe durch diese diffundieren können.

Insbesondere kann vorgesehen sein, dass die Stirnwand insbesondere eine konstante Dicke aufweist. Hierdurch wird eine besonders genaue Messung ermöglicht.

Zur Erhöhung der Stabilität sowie der Standfestigkeit der Abdeckkappe kann vorgesehen sein, dass eine Grundwand, die die Mantelwand an dem von der Stirnwand abgewandten Ende, insbesondere einstückig, fortsetzt, und die vorzugsweise parallel oder in einem Winkel von höchstens 10° zur Stirnwand von der Mantelwand nach außen, insbesondere mit ansteigendem Normalabstand zur Stirnwand, verläuft, wobei die Grundwand vorzugsweise kreisringförmig ausgebildet ist und insbesondere einen Außenradius zwischen 2 mm und 50 mm und/oder einen Innenradius zwischen 1 mm und 45 mm aufweist und/oder eine Kreisringbreite von 0,5 mm bis 35 mm aufweist.

Eine besonders vorteilhafte Anpassung an Messgeräte mit einer Auswurfvorrichtung oder einem Auswurfelement sieht vor, dass die Mantelwand im Bereich der Umfangskante der Stirnwand in einem Winkel von zwischen 20° und 95°, insbesondere zwischen 60° und 80°, zur Stirnwand nach außen absteht, wobei sich die Mantelwand, insbesondere konisch, erweitert.

Eine vorteilhafter Aspekt zur Verbindung der Abdeckkappe mit einem Messgerät sieht vor,

- dass die Abdeckkappe in dem der Stirnwand abgewandten Ende der Mantelwand Rastausnehmungen und/oder Rastvorsprünge zur lösbaren Befestigung an einer Messvorrichtung vorgesehen sind, und/oder
- dass insbesondere die einzelnen Rastausnehmungen und/oder Rastvorsprünge jeweils denselben Normalabstand zur Stirnwand aufweisen und/oder in Umfangsrichtung der Mantelwand gleichmäßig verteilt sind, und/oder wobei die Gesamtzahl der Rastausnehmungen und/oder Rastvorsprünge zwei oder vier beträgt, und/oder
- dass insbesondere die Rastausnehmungen und/oder Rastvorsprünge in einem Winkel von 5° bis 40° und/oder höchstens 2 mm, insbesondere höchstens 0,3 mm von der Mantelwand abstehen oder in diese eindringen. Hierdurch wird es zusätzlich ermöglicht, die Abdeckkappe einfach und automatisiert vom Messgerät zu entfernen.

Ein weiterer bevorzugter Aspekt der Erfindung sieht vor, dass die Stirnwand strahlungs-, dampf-, feuchtigkeits-, partikel-, und/oder lichtdurchlässig ausgebildet ist und/oder eine Anzahl von, insbesondere dampfdurchlässigen oder gasdurchlässigen, Mikrolöchern aufweist. Hierdurch wird es möglich, die so ausgebildete Abdeckkappe für eine Vielzahl unterschiedlicher Messgeräte anwendbar zu machen.

Eine besonders anpassungsfähige, robuste und widerstandsfähige Weiterbildung der Erfindung sieht vor, dass die Abdeckkappe materialeinheitlich und/oder einstückig ausgebildet ist, und insbesondere aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe (10), insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe einen Farbstoff aufweist und die Stirnwand für bestimmte, insbesondere sichtbare, Wellenlängenanteile undurchsichtig oder absorbierend ausgebildet ist.

Eine besonders einfach herzustellende Abdeckkappe, die ein einfaches Zusammenfalten bzw. Zusammenklappen ermöglicht, sieht vor, dass die Dicke der Mantelwand von der Stirnwand zu dem von der Stirnwand fernen Ende der Mantelwand hin, insbesondere zumindest über einen Teilbereich der Mantelwand, vorzugsweise kontinuierlich, zunimmt. Eine im Bereich der Stirnwand besonders robuste Abdeckkappe, die ein einfaches Hantieren beim Aufsetzen auf das Messgerät ermöglicht, sieht vor, dass die Mantelwand im Nahebereich der Stirnwand einen, insbesondere an die Umfangskante anschließenden, ersten Verdickungsabschnitt aufweist, in dem die Dicke der Mantelwand die Dicke der Stirnwand übersteigt,
dass die Mantelwand einen an den ersten Verdickungsabschnitt anschließenden Zwischenabschnitt aufweist, wobei die Dicke der Mantelwand im Zwischenabschnitt geringer ist als die Dicke der Mantelwand im ersten Verdickungsabschnitt,

dass die Mantelwand einen an den Zwischenabschnitt anschließenden und bis zu dem der Stirnwand fernen Ende der Mantelwand reichenden zweiten Verdickungsabschnitt aufweist, und

dass die Dicke der Mantelwand im zweiten Verdickungsabschnitt größer ist als die Dicke der Mantelwand im Zwischenabschnitt.

Eine einfache Fertigung wird dadurch ermöglicht, dass die Dicke der Mantelwand, insbesondere in axialer oder radialer Richtung, einen über die Mantelwand kontinuierlichen, knick- und kantenfreien Verlauf aufweist, und/oder als Dicke die jeweils mittlere Dicke im jeweiligen Abschnitt der Mantelwand gilt, und/oder

dass die Mantelwand, insbesondere mit Ausnahme des Bereichs der Rastvorsprünge und/oder Rastausnehmungen rotationssymmetrisch ausgebildet ist.

Besonders vorteilhafte Ausprägungen und Weiterbildungen der Erfindung sehen vor, dass die Dicken des ersten Verdickungsabschnitts, des Zwischenabschnitts und des zweiten Verdickungsabschnitts zueinander im folgenden Verhältnis stehen:

$$2 < d_1 : d_2 < 5 \text{ und/oder } 2 < d_3 : d_2 < 5 \text{ und/oder } 0,8 < d_1 : d_3 < 1,25$$

und/oder dass die Dicken des ersten Verdickungsabschnitts, des Zwischenabschnitts und des zweiten Verdickungsabschnitts wie folgt festgelegt sind:

150 $\mu$m < $d_1$ < 250 $\mu$m und/oder 50 $\mu$m < $d_2$ < 100 $\mu$m und/oder 150 $\mu$m < $d_3$ < 250 $\mu$m.. Hierdurch wird eine besonders vorteilhafte Abdeckkappe ausgebildet, die in ihrer der Stirnfläche fernen Endbereich besonders stabil und robust ist, im Bereich der Stirnfläche eine erhöhte Stabilität und im Zwischenbereich zwischen den beiden Verdickungsabschnitten eine gute Elastizität und Komprimierbarkeit aufweist.

Eine besonders vorteilhafte Auswahl der einzelnen Höhen der Verdickungsabschnitte, sowie des Zwischenabschnitts sieht vor, dass die Höhe des ersten Verdickungsabschnitts, die Höhe des Zwischenabschnitts sowie die Höhe des zweiten Verdickungsabschnitts in folgendem Verhältnis stehen:

$$0,2 < h_1 : h_2 < 0,6 \text{ und/oder } 0,8 < h_3 : h_2 < 1,25 \text{ und/oder } 0,2 < h_1 : h_3 < 0,6.$$

Zur Abdeckung typischer Messgeräte ist es im vielen Fällen vorteilhaft, wenn die Mantelwand eine Höhe von 2mm bis 80mm aufweist und/oder

- dass das Verhältnis zwischen der Höhe und der maximalen Abmessung, insbesondere der Durchmesser oder die Diagonale, der Stirnwand (1) zwischen 0,01 und 55 liegt.

Um ein für den zu vermessenden Gegenstand oder für die zu vermessende Person besonders schonendes Messen zu ermöglichen, kann vorgesehen sein, dass die Stirnwand kreisförmig ausgebildet ist und insbesondere einen Durchmesser von 1 mm bis 50 mm aufweist, oder dass die Stirnwand quadratisch oder rechteckig

- ausgebildet ist und wobei die Seitenkanten der Stirnwand eine Länge von zwischen 2 mm und 40 mm aufweisen.

Weiters betrifft die Erfindung ein Messgerät insbesondere zur Hautanalyse, das mit einer Abdeckkappe mit Rastausnehmungen und/oder Rastvorsprüngen abdeckbar ist. Insbesondere ist das Messgerät mit einer erfindungsgemäßen Abdeckkappe abdeckbar und mit diesem verrastbar. Das Messgerät umfasst ein Gehäuse und einen darin angeordneten Sensor, wobei das Gehäuse einen konisch zulaufenden Gehäuseteil mit einer vorderen Stirnfläche aufweist, an dem eine Anzahl von Rastausnehmungen und/oder Rastvorsprüngen zum Verrasten mit den Rastausnehmunge und/oder Rastvorsprüngen der Abdeckkappe angeordnet ist,

insbesondere derart, dass im eingerasteten Zustand die Stirnwand der Abdeckkappe an der vorderen Stirnfläche anliegt. Mit diesem Messgerät ist es sehr einfach möglich, Abdeckkappen hygienisch und einfach durch Auswerfen bzw. Wegschießen der Abdeckkappe in einen dafür vorgesehenen Behälter zu entsorgen. Insbesondere ist es nicht erforderlich, die Abdeckkappe in dem Bereich zu berühren, mit dem sie auf dem zu vermessenden Gegenstand, oder auf dem zu vermessenden Lebewesen bei der Messung aufgesetzt war.

Gemäß einer baulich vorteilhaften Ausgestaltung zum Auswerfen der Abdeckkappe ist ein Auswurfelement vorgesehen, das in dem der vorderen Stirnwand entfernten Ende des konisch zulaufenden Gehäuseteils angeordnet ist und das von der vorderen Stirnwand weiter entfernt ist als die Rastausnehmungen und/oder Rastvorsprüngen,

- wobei das Auswurfelement zum Schieben einer eingerasteten Abdeckkappe durch Druckbeaufschlagung der Abdeckkappe in Richtung der vorderen Stirnwand ausgebildet ist und/oder
- wobei das Auswurfelement auf dem Gehäuse in Richtung der vorderen Stirnwand verschiebbar gelagert ist.

Um ein definiertes Auswerfen und Entsorgen der Abdeckkappe mit dem Messgerät zu erzielen, kann vorgesehen sein, dass an das Auswurfelement ein Hebelfortsatz anschließt oder von dem Auswurfelement ein Hebelfortsatz abgeht oder das Auswurfelement mit einem Hebelfortsatz verbunden ist,

- wobei der Hebelfortsatz in seinem Mittenbereich schwenkbar mit dem Gehäuse verbunden ist, sodass sich ein zweiarmiger Hebel ergibt, an dessen einen Ende das Auswurfelement angeordnet ist,
- wobei der am Gehäuse angelenkte Hebelfortsatz bei seinem Verschwenken, das Auswurfelement in Richtung der vorderen Stirnwand drückt.

Um ein vollständig berührungsloses Entfernen der Abdeckkappe vom Messgerät zu ermöglichen, kann vorgesehen sein, dass ein Auswurfelement, das in dem der vorderen Stirnwand entfernten Ende des konisch zulaufenden Gehäuseteils angeordnet ist und das von der vorderen Stirnwand weiter entfernt ist als die Rastausnehmungen und/oder Rastvorsprüngen, und

- dass das Auswurfelement zum Schieben einer eingerasteten Abdeckkappe durch Druckbeaufschlagung der Abdeckkappe in Richtung der vorderen Stirnwand ausgebildet ist und/oder
- dass das Auswurfelement auf dem Gehäuse in Richtung der vorderen Stirnwand (22) verschiebbar gelagert ist. Hierbei ist ein händisches Entfernen der Abdeckkappe vom Messgerät überhaupt nicht erforderlich und kann vielmehr durch Betätigung des Auswurfelements erfolgen.

Besonders einfach kann das Auswerfen erfolgen, wenn das Auswurfelement ringförmig, insbesondere kreisringzylinderförmig, ausgebildet ist und den konisch zulaufenden Gehäuseteil umgibt.

Eine besonders vorteilhafte Betätigung des Auswurfelements sieht vor, dass an das Auswurfelement ein Hebelfortsatz anschließt oder von dem Auswurfelement ein Hebelfortsatz abgeht oder das Auswurfelement mit einem Hebelfortsatz verbunden ist, wobei der Hebelfortsatz in seinem Mittenbereich schwenkbar mit dem Gehäuse verbunden ist, sodass sich ein zweiarmiger Hebel ergibt, an dessen einen Ende das Auswurfelement angeordnet ist, wobei insbesondere an dessen anderen Ende ein Betätigungselement vorgesehen ist, wobei der am Gehäuse angelenkte Hebelfortsatz bei seinem Verschwenken, insbesondere durch Kraftbeaufschlagung des Betätigungselements, das Auswurfelement in Richtung der vorderen Stirnwand drückt.

Hierbei kann zur weiteren Verbesserung der Bedienbarkeit und Benutzbarkeit vorgesehen sein, dass das Auswurfelement und/oder der Hebelfortsatz durch ein Federelement mit einer Kraft oder Vorspannung beaufschlagt ist, die das Auswurfelement von der vorderen Stirnwand weg drückt oder weg zieht.

Ein besonders einfaches und wirkungsvolles Auswerfen der Abdeckkappe sieht vor, dass die einzelnen Rastausnehmungen und/oder Rastvorsprünge jeweils denselben Normalabstand zur Stirnfläche aufweisen und/oder in Umfangsrichtung der Mantelwand gleichmäßig verteilt sind, und/oder die Gesamtzahl der Rastausnehmungen und/oder Rastvorsprünge zwei oder vier beträgt, und/oder

dass die Rastausnehmungen und/oder Rastvorsprünge in einem Winkel von 10° bis 30° und/oder höchstens 1 mm, insbesondere höchstens 0,5 mm, von der Mantelwand abstehen und/oder

dass zwei Rastausnehmungen und/oder Rastvorsprünge vorgesehen sind, die denselben Abstand zu einem Punkt, in dem Bereich aufweisen, an dem der Hebelfortsatz an das Auswurfelement anschließt oder von diesem abgeht.

Eine Abdeckkappe mit einer besonders dünnen Stirnwand kann mit dem erfindungsgemäßen Verfahren hergestellt werden. Hierbei ist vorgesehen,

a) dass eine Kunststofffolie, insbesondere eine Folie bestehend aus PETG (Polyethylenterephthalat Glycol), als Ausgangsmaterial für einen Tiefziehprozess herangezogen wird, die vorzugsweise eine Dicke zwischen 0,04 mm und 0,5 mm aufweist,
b) dass ein Stempel mit einer Anzahl von separat beweglichen, aneinander anliegenden Teilstempeln auf die Kunststofffolie gepresst wird, wobei die einzelnen Teilstempel normal zur Ebene der Kunststofffolie bewegt werden und wobei die Kunststofffolie (31) durch den Druck der Teilstempel in eine Gegenform gedrückt wird und zu einer Kappenform verformt wird,
c) dass die einzelnen Teilstempel des Stempels während oder nach Schritt b) zueinander beabstandet werden, wobei jeder der Teilstempel von der Position des Stempels aus radial nach außen bewegt wird, wodurch in der Kunststofffolie eine Stirnwand, insbesondere mit einer Dicke im Bereich von weniger als 100 $\mu$m, vorzugsweise zwischen 3 $\mu$m und 20 $\mu$m, insbesondere zwischen 5 $\mu$m und 10 $\mu$m, ausgebildet wird.

Die Erfindung verwendet eine Abwandlung eines Tiefziehverfahrens, um besonders dünne Wandstärken der Stirnwand zu erreichen. Insbesondere die rotatorische Komponente der Ausformung der Abdeckkappen ermöglicht die Ausbildung sehr dünner Wandstärken.

Zur Ausbildung von Abdeckkappen mit unterschiedlichen Stärken der Mantelwand kann vorgesehen sein, dass anschließend an Schritt c) die Teilstempel aus der ausgebildeten Kappenform entfernt und ein weiterer Stempel mit einer ebenen Stirnfläche und einem sich erweiternden Stempelkörper, in das durch die Teilstempel ausgeformten Volumen der Kunststofffolie eingebracht wird, wobei die Kunststofffolie zwischen dem weiteren Stempel und einer zweiten Gegenform in eine Kappenform gebracht wird,

wobei vorzugsweise die Kunststofffolie, der weitere Stempel oder die zweite Gegenform nach dem Erhitzen der Kunststofffolie auf eine Temperatur zwischen 50°C und 90°C vorgeschoben wird.

Um die Wandstärken unterschiedlich ausbilden zu können, kann vorgesehen sein, dass zur Einstellung der jeweiligen Dicke der von der Stirnwand abgehenden Mantelwand die Vorschubgeschwindigkeit und/oder Temperatur der Teilstempel, des weiteren Stempels und/oder die Temperatur der Kunststofffolie und/oder die Temperatur der ersten oder zweiten Gegenform eingestellt wird,

wobei zur Verringerung der Wandstärke im Randbereich der durch den weiteren Stempel gebildeten Ausnehmung eine Erhöhung der genannten Temperaturen sowie eine Erhöhung der Vorschubgeschwindigkeit vorgenommen wird und wobei zur Erhöhung der Wandstärke im Randbereich der durch den weiteren Stempel gebildeten Ausnehmung eine Verringerung der genannten Temperaturen sowie eine Verringerung der Vorschubgeschwindigkeit vorgenommen wird.

Eine besonders vorteilhafte Abdeckkappe, die nach ihrer Verwendung gut zusammenfaltbar ist und vorteilhaft von einem Messgerät entfernt werden kann, kann hergestellt werden, indem die Temperatur des weiteren Stempels und/oder der Kunststofffolie sowie die Vorschubgeschwindigkeit des weiteren Stempels derart eingestellt werden,

dass während eines ersten Vorschubzeitraums des weiteren Stempels ein Abschnitt der Mantelwand mit einer vorgegebenen Dicke, insbesondere von 50 μm bis 100 μm, ausgebildet wird, und

dass während eines dem ersten Vorschubzeitraums nachfolgenden zweiten Vorschubzeitraums des weiteren Stempels ein Abschnitt der Mantelwand mit einer vorgegebenen Dicke, insbesondere von 150 μm und 250 μm, ausgebildet wird, die dicker ist als die Dicke der im ersten Vorschubzeitraum erstellten Mantelwand.

Hierbei kann vorteilhaft vorgesehen sein, dass die Wanddicke während des ersten Vorschubzeitraums des weiteren Stempels auf einen Wert eingestellt wird, der dem Doppelten bis Fünffachen der Wanddicke desjenigen Mantelbereichs entspricht, der bei der Ausformung der Stirnwand durch das Einbringen der Teilstempel ausgeformt wurde.

Zur Erstellung von Abdeckkappen mit kreisförmigen Stirnwänden kann vorgesehen sein, das dass die Teilstempel während ihrer radial von ihrem Zentrum weg gerichteten Bewegung in Schritt c) entlang einer um eine zentrale Achse spiralförmig nach außen verlaufenden und sich aufweitenden Kurve rotierend bewegt werden.

Eine besonders anpassungsfähige, robuste und widerstandsfähige Weiterbildung der Erfindung sieht vor, dass die Kunststofffolie aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe, insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe einen Farbstoff aufweist und die Stirnwand für bestimmte Wellenlängenanteile undurchsichtig oder absorbierend ausgebildet wird.

Zur Ausbildung von Merkmalen, insbesondere von Rastausnehmungen, im Innenbereich der Mantelfläche sowie auf der Stirnfläche kann vorgesehen sein, dass nach der teilweisen oder vollständigen Ausformung der Mantelwand die Teilstempel oder der weitere Stempel zurückgefahren werden und das ausgeformte Volumen mit, insbesondere erhitzter Druckluft, beaufschlagt wird und dass der weitere Stempel anschließend gegebenenfalls in das ausgeformte Volumen zur Durchführung des nächsten Ausformungsschritt eingebracht wird,

wobei insbesondere durch Beaufschlagung mit Druckluft Rastausnehmungen im Innenmantel der Mantelwand ausgeformt werden.

Um die erstellten Abdeckkappen vom Körper der Kunststofffolie zu entfernen, kann vorgesehen sein, dass nach der Ausformung der Mantelwand die an die Mantelwand anschließende verbleibende Kunststofffolie von der Mantelwand entlang einer vorgegebenen Schnittlinie abgetrennt wird, wobei die Schnittlinie insbesondere so gewählt wird, dass ein Teilabschnitt der an die Mantelwand anschließenden verbleibenden Kunststofffolie innerhalb der Schnittlinie liegt und eine Grundwand ausbildet.

Um die Durchlässigkeit der Folie festzulegen, kann vorgesehen sein, dass die Kunststofffolie, insbesondere nach ihrer Ausformung, für einen vorgegebenen Zeitraum, insbesondere für zumindest 3 Sekunden, auf ein vorgegebene Temperatur zwischen 30°C bis 120°C, insbesondere auf eine Temperatur zwischen 50°C und 90°C,aufgewärmt wird, und/oder

dass die Teilstempel und/oder der weitere Stempel und/oder die verwendeten Gegenformen vor der Bearbeitung der Kunststofffolie auf eine Temperatur zwischen 30°C bis 120°C, insbesondere auf eine Temperatur zwischen 50°C und 90°C, erwärmt werden und während der Bearbeitung auf einer solchen Temperatur gehalten werden.

Hierbei kann insbesondere vorgesehen sein, dass der die Kunststofffolie bildende Kunststoff durch die Erwärmung umgeformt wird, wobei dieser insbesondere seine thermoplastischen Eigenschaften verliert und/oder wobei vorzugsweise durch die Wärmeeinwirkung die die Kunststofffolie bildenden Molekülketten aufgespalten werden.

Um eine verbesserte Diffusion von Stoffen durch die Stirnwand zu ermöglichen, kann vorgesehen sein, dass, insbesondere mittels eines Lasers oder einer erhitzten Mikronadel oder Nanonadel oder mittels Ätzen, Löcher, insbesondere Mikrolöcher oder Nanolöcher, in der Stirnwand ausgebildet werden.

Mehrere Ausführungsbeispiele der Erfindung werden anhand der folgenden Zeichnungsfiguren dargestellt.

**Fig. 1a** zeigt eine erste Ausführungsform einer erfindungsgemäßen Abdeckkappe im Querschnitt. **Fig. 1b** zeigt die in **Fig. 1a** dargestellte Abdeckkappe von der Seite. **Fig. 2** zeigt eine Abdeckkappe gemäß einer besonders einfachen Ausführungsform der Erfindung im Querschnitt.

**Fig. 3** zeigt ein erfindungsgemäßes Messgerät. **Fig. 4** zeigt ein erfindungsgemäßes Messgerät mit aufgesetzter Abdeckkappe. **Fig. 4a** zeigt ein den Auswurf der Abdeckkappe vom Messgerät.

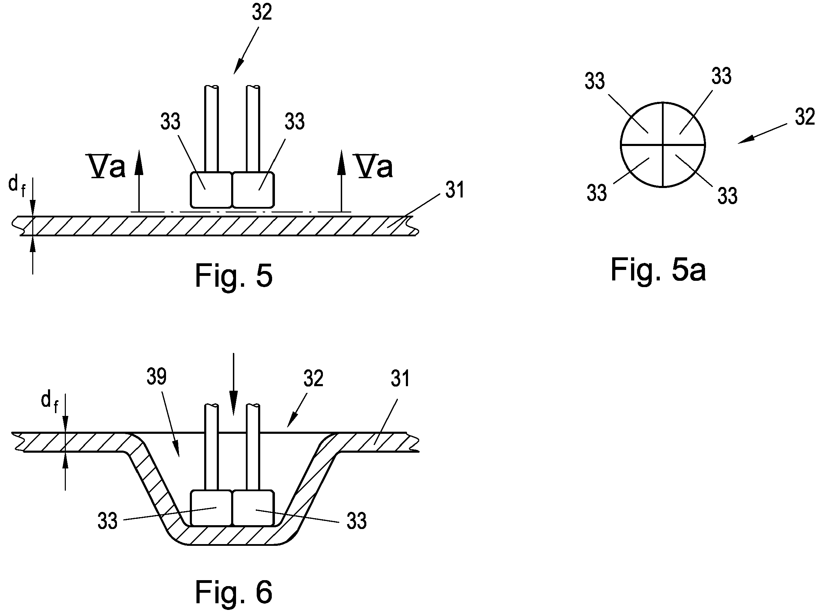

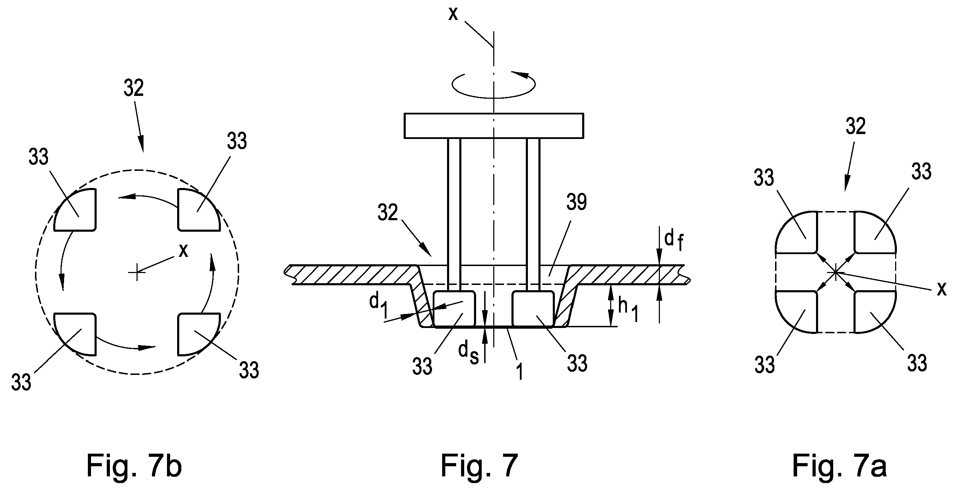

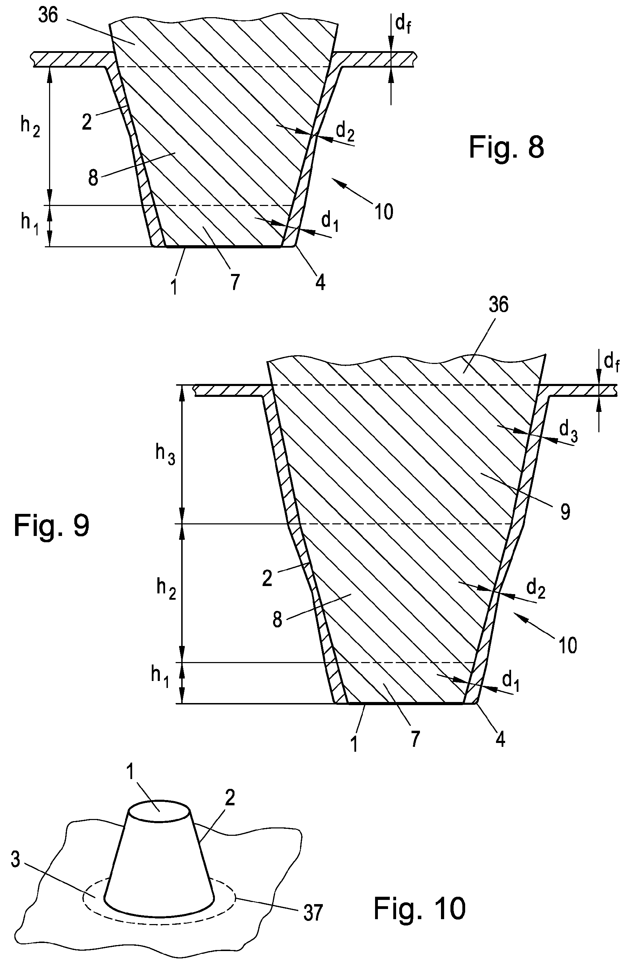

**Fig. 5** zeigt eine Kunststofffolie mit der die Abdeckkappe ausgebildet wird. **Fig. 5a** zeigt die Stirnfläche des Stempels und seiner Teilstempel. **Fig. 6** zeigt das Vordringen der Teilstempel in die Kunststofffolie. **Fig. 7** zeigt das Aufweiten der Teilstempel und die Ausbildung der Stirnfläche der Abdeckkappe. **Fig. 7a** zeigt die Bewegung der Teilstempel zur Ausformung einer rechteckigen Stirnfläche. **Fig. 7b** die Bewegung der Teilstempel zur Ausformung einer kreisförmigen Stirnfläche. **Fig. 8 und 9** zeigen die Ausbildung unterschiedlich dicker Mantelwandabschnitte der Abdeckkappe. **Fig. 10** zeigt die Fertigstellung der Abdeckkappe durch Abtrennen von der Kunststofffolie.

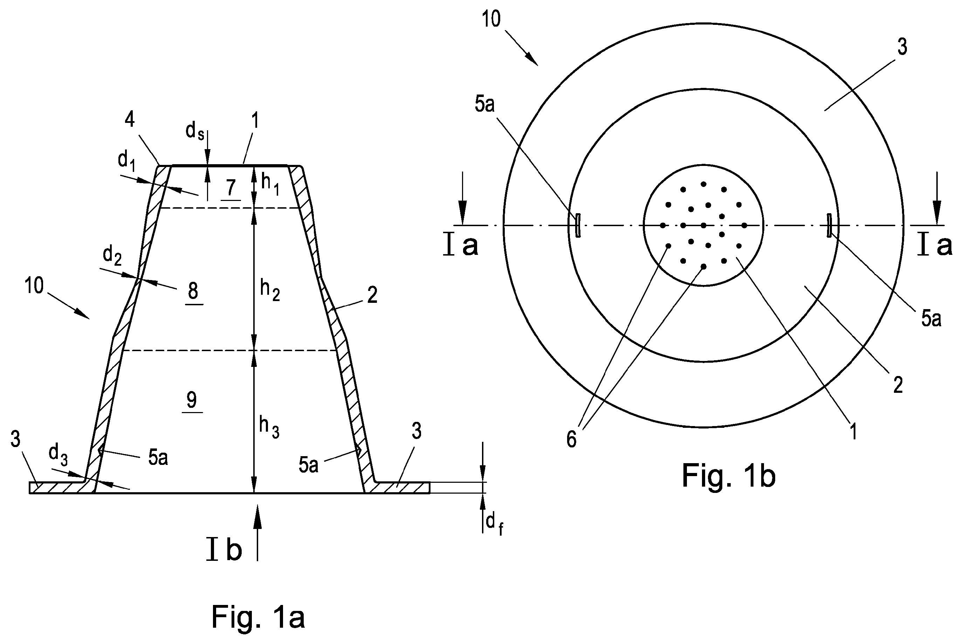

In **Fig. 1a** ist eine erste Ausführungsform einer erfindungsgemäßen Abdeckkappe 10 im Querschnitt (Schnittlinie **I a** in **Fig. 1b**) dargestellt. Dieselbe Abdeckkappe 10 ist in **Fig. 1b** von unten gemäß der in **Fig. 1a** angedeuteten Schnittlinie **I b** dargestellt. Diese Abdeckkappe 10 weist eine kreisrunde Stirnwand 1 auf, die von einer Umfangskante 4 begrenzt ist. An der Umfangskante 4 der Stirnwand 1 schließt eine Mantelwand 2 an, die an ihrem der Stirnwand 1 abgewandten Ende von einer Grundwand 3 fortgesetzt wird. Die Mantelwand 2 ist im Folgenden Ausführungsbeispiel sich annähernd konisch erweiternd ausgebildet und steht im Bereich der Umfangskante 4 in einen Winkel $\alpha$ von 75° von der Stirnwand 1 nach außen ab.

Die Stirnwand 1 weist eine sehr geringe Wanddicke $d_S$ von 10$\mu$m auf, vorteilhafterweise kann diese die Wanddicke $d_S$ in einem Bereich zwischen 0,05 $\mu$m und 20 $\mu$m, insbesondere in einem Bereich von 3 $\mu$m bis 20 $\mu$m liegen. Die Stirnwand 1 weist im vorliegenden Fall einen Durchmesser von 5 mm auf. Grundsätzlich kann dieser Durchmesser in einem sehr weiten Bereich liegen, insbesondere vorteilhaft sind Durchmesser im Bereich zwischen 2 mm und 20 mm, wobei, je nach Anwendungsfalls, auch größere Durchmesser von bis zu 100 mm durchaus Anwendung finden können.

Eine alternative, nicht dargestellte Ausführungsform der Erfindung weist an Stelle eines kreisförmigen Aufbaus der Stirnwand 1 eine quadratische Stirnwand 1 auf, die im Wesentlichen eine gleichgroße Wandfläche aufweist, wie in dem in **Fig. 1** dargestellten Ausführungsbeispiel und weist eine Kantenlänge von etwa 10 mm auf. Alternativ kann die Stirnwand 1 auch rechteckig, quadratisch oder elliptisch ausgebildet sein, wobei die Seitenkanten der Stirnwand 1 etwa eine Länge zwischen 2 mm und 40 mm, insbesondere zwischen 5 mm und 8 mm, aufweisen können.

In dem in **Fig. 1a** dargestellten Ausführungsbeispiel ist der Mantel 2 der Abdeckkappe 10 dreiteilig ausgebildet und umfasst im Nahebereich der Stirnwand 1 einen an die Umfangskante 4 anschließenden, ersten Verdickungsabschnitt 7 mit einer Dicke $d_1$, im vorliegenden Ausführungsbeispiel wurde eine Dicke $d_1$ im Bereich zwischen 150 und 250 $\mu$m für den ersten Verdickungsabschnitt gewählt. Die Dicke $d_1$ der Mantelwand 2 im ersten Verdickungsabschnitt 7 übersteigt die Dicke $d_S$ der Stirnwand 1 und beträgt im vorliegenden Ausführungsbeispiel 5 $\mu$m bis 8 $\mu$m. Der erste Verdickungsabschnitt 7 ist umlaufend ausgebildet, wobei der erste Verdickungsabschnitt 7 auf dem von der Stirnwand 1 fernen Ende kreisförmig begrenzt ist, und wobei in diesem Begrenzungsbereich ein Zwischenabschnitt 8 an den ersten Verdickungsabschnitt 7 anschließt.

Im Zwischenabschnitt 8 weist die Mantelwand 2 eine Dicke $d_2$ auf, die geringer ist als die Dicke $d_1$ der Mantelwand 2 im ersten Verdickungsabschnitt 7. Im vorliegenden Ausführungsbeispiel wird als Dicke $d_2$ der Mantelwand 2 im Zwischenabschnitt 8 eine Dicke von 50 bis 150 $\mu$m gewählt. Der Zwischenabschnitt 8 ist auf seinem dem ersten Verdickungsabschnitt 7 fernen Ende kreisförmig begrenzt, wobei an diese kreisförmige Begrenzung ein zweiter Verdickungsabschnitt 9 anschließt, in dem die Dicke $d_3$ der Mantelwand 2 größer ist als die Dicke $d_2$ der Mantelwand 2 im Zwischenabschnitt 8. Im vorliegenden Ausführungsbeispiel wurde eine Dicke $d_3$ im Bereich zwischen 150 und 250 $\mu$m für den ersten Verdickungsabschnitt gewählt. Der zweite Verdickungsabschnitt 9 schließt im vorliegenden Fall an das der Stirnwand 1 ferne Ende der Mantelwand 2 an, insbesondere grenzt der zweite Verdickungsabschnitt 9 unmittelbar an die Grundwand 3 an.

Durch diese spezielle Ausbildung weist die Mantelwand 2 einen umlaufenden Abschnitt 8 in Form des Zwischenabschnittes 8 auf, in dem die Dicke $d_2$ geringer ist als die Dicke $d_3$ der Mantelwand 2 im Bereich des von der Stirnwand 1 abgewandten Endes der Mantelwand 2. Die Dicke der Mantelwand 2 nimmt in axialer bzw. radialer Richtung, ausgehend von der Dicke $d_1$ im ersten Verdickungsbereich 7 stetig bzw. kontinuierlich ab und erreicht im Zwischenabschnitt 8 ein Minimum, anschließend nimmt die Dicke der Mantelwand 2 im zweiten Verdickungsbereich 9 bis zu den der Stirnwand 1 fernen Ende der Mantelfläche zu.

Die Dicke der Mantelwand 2 weist einen über die Mantelwand 2 kontinuierlichen, knick- und kantenfreien Verlauf auf. Soweit für die folgenden Betrachtungen notwendig, wird jeweils als Dicke der Mantelwand 2 im jeweiligen Abschnitt 7, 8, 9 der Mantelwand 2 die mittlere Dicke in diesem Abschnitt verstanden.

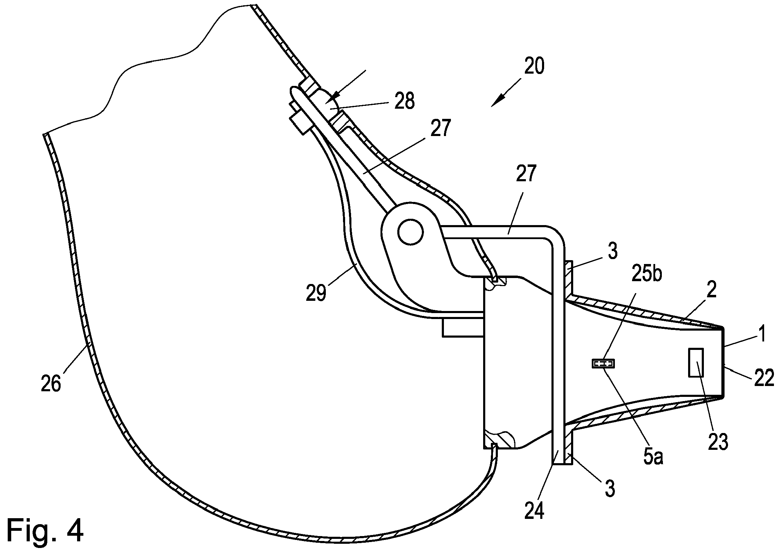

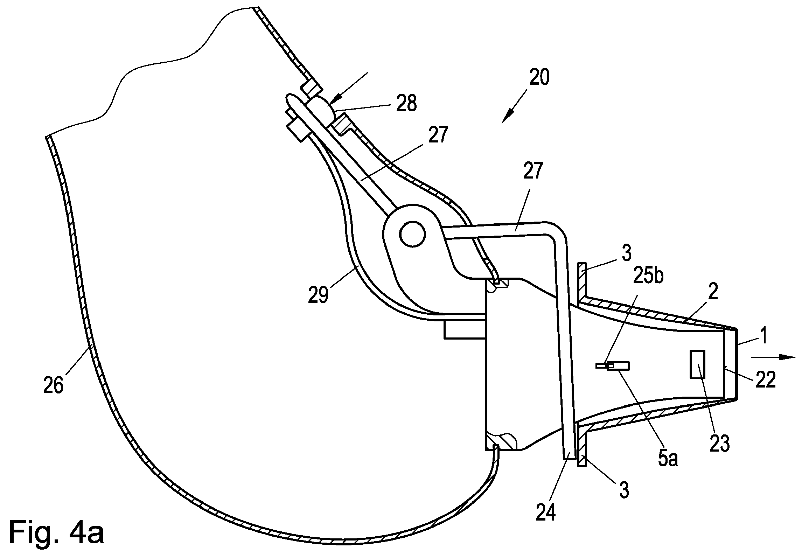

Um eine besonders vorteilhafte Anpassung der Stirnwand 1 an die Stirnfläche 21 (Fig. 4) eines Messgerätes 20 zu ermöglichen, ist die Dicke der Mantelwand 2 im Bereich des Zwischenabschnittes 8 dünner ausgebildet als in den übrigen beiden Verdickungsabschnitten 7, 9. Das Verhältnis der Dicke $d_2$ im Zwischenabschnitt 8 zur Dicke $d_1$ im ersten Verdickungsabschnitt 7 liegt etwa zwischen 2 und 5. Das Verhältnis der Dicke $d_2$ im Zwischenabschnitt 8 zur Dicke $d_3$ im zweiten Verdickungsabschnitt 9 liegt etwa zwischen 2 und 5. Die Dicke $d_1$ der Mantelfläche 2 im ersten Verdickungsabschnitt sowie die Dicke $d_3$ der Mantelfläche 2 im zweiten Verdickungsabschnitt 9 ist ungefähr gleichgroß. Das Verhältnis zwischen diesen beiden Dicken $d_1$, $d_3$ liegt etwa zwischen den Werten 0,8 und 1,25.

Im vorliegenden Ausführungsbeispiel weist die Mantelwand 2 als mittlere Dicke in den einzelnen Abschnitten 7, 8, 9 die folgenden Werte auf:

$$d_1 = 0{,}18\,\text{mm} \quad d_2 = 0{,}08\,\text{mm} \quad d_3 = 0{,}22\,\text{mm}$$

Die Höhe h der Mantelwand 2 beträgt im vorliegenden Ausführungsbeispiel 20 mm, sie kann jedoch, abhängig von der Form und vom Aufbau des abzudeckenden Messgerätes 20, unterschiedliche Werte aufweisen, die typischerweise zwischen 15 mm und 35 mm betragen. Das Verhältnis zwischen der Höhe h der Mantelwand 2 und der maximalen Abmessung der Stirnwand 1 liegt im Bereich zwischen 0,02 und 5.

Der erste Verdickungsabschnitt 7 ist im Bereich der Stirnwand 1 ausgebildet und weist im vorliegenden Ausführungsbeispiel eine Höhe von etwa 4 mm auf. Die übrigen beiden Abschnitte der Mantelwand 2, nämlich der Zwischenabschnitt 8 sowie der zweite Verdickungsabschnitt 9 weisen in etwa dieselbe Höhe auf, die im vorliegenden Fall jeweils ca. 10 mm beträgt. Typischerweise stehen die Höhe $h_1$ des ersten Verdickungsabschnittes 7, die Höhe $h_2$ des Zwischenabschnittes 8 sowie die Höhe $h_3$ des zweiten Verdickungsabschnittes 9 in folgendem Verhältnis zueinander:

$$0{,}2 < h_1 : h_2 < 0{,}6; \quad 0{,}8 < h_3 : h_2\ 1{,}25; \quad 0{,}2 < h_1 : h_3 < 0{,}6$$

Eine besondere weitere Ausgestaltung der Erfindung ermöglicht eine verbesserte Handhabung sowie eine geringere Riss- oder Bruchanfälligkeit der Abdeckkappe 20 beim Hantieren. Um eine bessere Handhabung zu gewährleisten, wird in der Abdeckkappe 10 im Bereich des zweiten Verdickungsabschnittes 9 bzw. in dessen unterem, der Stirnwand 1 fernem Endbereich ein Versteifungselement angeordnet. Ein solches Versteifungselement ist mit dem Innen- oder Außenmantel der Mantelfläche 2 verbunden und weist die Form einer Manschette auf, deren Oberflächenverlauf dem Oberflächenverlauf des Innen- oder Außenmantels entspricht. Diese Manschette besteht vorzugsweise aus versteiftem Kunststoff bzw. einem Kunststoff, der eine höhere innere Steifigkeit aufweist als der die Abdeckkappe 10 ausbildende Kunststoff.

Die Grundwand 3, die die Mantelwand 2 an dem von der Stirnwand 1 abgewanden Ende fortsetzt, verläuft im vorliegenden Ausführungsbeispiel parallel zur Stirnwand 1. Alternativ könnte die Grundwand 3 jedoch auch in einem Winkel von 10° zur Stirnwand 1 nach außen sowie von der Stirnwand 1 weg verlaufen. Vorzugsweise steht die Grundwand 3 von der Mantelwand 2 nach außen ab, wobei mit zunehmendem radialen Abstand der Normalabstand der Grundwand 3 von der Stirnwand ansteigt.

Die Grundwand 3 ist im vorliegenden Ausführungsbeispiel kreisringförmig und parallel zur Stirnwand 1 ausgebildet und weist einen Außendurchmesser von 21 mm und einen Innendurchmesser von 18 mm auf. Die Kreisringbreite beträgt im vorliegenden Fall 1,5 mm.

Weiters weist die Abdeckkappe 10 an der Innenseite der Mantelwand 2 Rastausnehmungen 5a auf, die eine lösbare Befestigung an einem Messgerät 20 (Fig. 4) ermöglichen. Im vorliegenden Ausführungsbeispiel sind zwei Rastausnehmungen 5b so angeordnet, dass sie einander an der Mantelwand der Abdeckkappe 10 gegenüber liegen. Alternativ können anstelle von Rastausnehmungen 5a auch nicht dargestellte Rastvorsprünge 5b vorgesehen sein. Grundsätzlich liegt der Zweck der Rastausnehmungen 5a und Rastvorsprünge 5b darin, eine Verrastung mit einem Messgerät 20 bzw. mit auf dem Messgerät 20 angeordneten Rastausnehmungen 25a und/oder Rastvorsprüngen 25b zu erzielen.

Die beiden Rastausnehmungen 5a sind im vorliegenden Ausführungsbeispiel als Ausnehmungen an der Innenseite der Mantelwand 2 im Bereich des zweiten Verdickungsabschnittes 9 ausgebildet und verlaufen etwa in einem Winkel $\beta_1$, $\beta_2$ von 20° gegenüber der Mantelwand 2. Die Rastausnehmungen 5a ragen etwa 60 $\mu$m in den zweiten Verdickungsbereich 9 hinein.

Alternativ besteht auch die Möglichkeit, anstelle von Rastausnehmungen 5b Rastvorsprünge 5a vorzusehen. Diese stehen von der Innenseite der Mantelwand 2 in einem Winkel $\beta_1$, $\beta_2$ von 10° bis 30° nach innen ab und stehen etwa 1,5 mm von der Mantelwand 2 nach innen ab. Die Rastvorsprünge 5a liegen einander auf derselben Höhe jeweils mit demselben Normalabstand zur Stirnwand 1 gegenüber und sind in Umfangsrichtung der Mantelwand 2 gleichmäßig verteilt, d.h. im vorliegenden Ausführungsbeispiel liegen sie einander um 180° gegenüber.

Alternativ besteht auch die Möglichkeit, dass die Rastausnehmungen 5a oder die Rastvorsprünge 5b in größerer Zahl vorhanden sind. Insbesondere erweist sich eine Anzahl von vier Rastvorsprüngen 5b oder Rastausnehmungen 5a als vorteilhaft; diese sind in Umfangsrichtung zueinander jeweils um 90° beabstandet.

In einer besonderen Ausführungsform der Erfindung kann vorgesehen sein, dass jeweils zwei der vier Rastelemente als Rastausnehmungen 5a, die übrigen als Rastvorsprünge 5b ausgebildet sind, wobei einander jeweils zwei Rastausnehmungen 5a und zwei Rastvorsprünge 5b um 180° umfangsseitig gegenüberliegen. Durch diese Maßnahme kann die Abdeckkappe 10 gegenüber einem Messgerät 20 (**Fig. 4**) vorteilhaft in ihrer Position gehalten werden, wobei die Mantelwand 2, insbesondere im Bereich des Zwischenabschnittes 8, einer elastischen Dehnung aufgrund der dünnen Dicke $d_2$ unterliegt und eine besonders gute Anpassung der Stirnwand 1 an eine abzudeckende Stirnfläche 21 (**Fig. 4**) des Messgerätes 20 ermöglicht wird. Das Ausmaß der Dehnung sowie die Dehnbarkeit der Mantelwand 2 sowie des Zwischenabschnitts 8 ist von dem verwendeten Kunststoff sowie von der Dicke $d_2$ der Mantelwand 2 im Bereich des Zwischenabschnitts 8 abhängig.

Weiters besteht auch die Möglichkeit, entweder am Messgerät 20 oder an der Abdeckkappe 10 umlaufende Rastelemente in Form von Rastvorsprüngen oder Rastrillen vorzusehen, wobei vorteilhafterweise ein umlaufendes Rastelement nur entweder auf der Abdeckkappe 10 oder auf dem Messgerät vorgesehen ist, um ein optimales Auswerfen der Abdeckkappe 10 vom Messgerät zu gewährleisten.

Im vorliegenden Ausführungsbeispiel ist die gesamte Abdeckkappe 10 materialeinheitlich ausgebildet und besteht aus Kunststoff. Im vorliegenden Fall wurde PETG verwendet, das mit einer geringen Menge an Farbstoffen vermischt ist. Die Verwendung von Farbstoffen für die Abdeckkappen 10 ermöglicht die Unterscheidung verschiedener Abdeckkappen mit unterschiedlichen Materialeigenschaften, insbesondere mit unterschiedlicher Dicke $d_S$ der Stirnwand 1. Es ist jedoch auch möglich, durch Beigabe von Farbstoffen die Stirnwand für bestimmte Wellenlängenanteile undurchsichtig oder absorbierend zu machen.

Anstelle von PETG (Polyethylenterephthalat Glycol) können auch aus dem Stand der Technik bekannte Kunststoffe, wie etwa Polyethylen oder Polypropylen oder Mischungen hieraus, verwendet werden.

In alternativen Ausführungsbeispielen der Erfindung die Abdeckkappe aus einer der folgenden Substanzen oder aus einer Mischung der folgenden Substanzen bestehen: PETG (Polyethylenterephthalat Glycol), PP (Polypropylen), PE (Polyethylen), PC (Polycarbonat), PVC (Polyvinylchlorid), PS (Polystyrol), ABS (Acrylonitrile butadiene styrene), HDPE (High Density Polyethylene), LDPE (Low Density Polyethylene), PET (Polyethylene terephthalate), PMMA (Polymethyl methacrylate), ECOTERM S 900 T1. Hierbei können dem Material der Abdeckkappe 10 insbesondere zusätzlich einer oder mehrere der folgenden Bestandteile zugesetzt sein: Additive, Stabilisatoren, Farbmittel, Füllstoffe, Verstärkungsstoffe, PETG / Copolyester 6763.

Durch die konkrete Auswahl der Kettenlänge der einzelnen Molekülketten des Polyethylens, Polypropylens oder PETG kann festgelegt werden, für welche chemischen Verbindungen die Stirnwand 1 durchlässig ist bzw. für welche chemischen Verbindungen die Stirnwand undurchlässig ist. So besteht etwa die Möglichkeit, bestimmte Proben wie Blut, Urin, Schweiß oder Kot sowie lebende Proben, beispielsweise Haut, zu vermessen, wobei nur bestimmte, für die Messung gewünschte Inhaltsstoffe durch die Stirnwand 1 diffundieren können, etwa Natrium, Kalium, Chlor, Magnesium Vitamine, Hormone, Glycose, Alkohol, Spurenelemente und Wasserdampf, andere Bestandteile der Probe jedoch nicht oder nur nach sehr langer Zeit im Bereich Stunden durch die Stirnwand 1 diffundieren können, wie etwa Wasser, Blut, Urin oder Kot. Durch die Wahl kürzerer Ketten entstehen Löcher im jeweiligen Kunststoff oder in der jeweiligen Kunststoffstruktur, sodass größere Moleküle durch die Stirnwand 1 diffundieren können. Je länger die Kettenmoleküle gewählt werden, desto engmaschiger verläuft die Kunststoffstruktur und desto kleiner müssen Moleküle sein, um die Stirnwand 1 durchdringen zu können. Weiters entscheidet auch die Dicke $d_S$ der Stirnwand 1 über die Durchlässigkeit, da Moleküle leichter durch eine dünnere Stirnwand 1 diffundieren können, als durch eine dickere Stirnwand 1. Sofern aufgrund von Vorgaben betreffend die Stabilität der Stirnwand 1 eine bestimmte Dicke erforderlich ist, können alternativ Mikrolöcher oder Nanolöcher 6 mit einem Durchmesser von 0,1 nm bis 5 µm, allenfalls bis 400 µm, in die Stirnwand 1, beispielsweise mit einer Mikronadel oder einem Laserstrahl, erstellt werden. Alternativ können auch mit chemischen Prozessen wie etwa durch Ätzen Löcher 6 in der Stirnfläche 1 erzielt werden. Um eine weitere Beschädigung der Stirnfläche 1 zu vermeiden, wird diese im Anschluss an die Erstellung der Löcher vom jeweiligen Ätzmittel gereinigt, insbesondere abgespült.

Da die Molekülgrösse von Wasser ist 0,3 nm und die Molekülgrösse von Wasserdampf ist 0,1 nm beträgt, kann durch Vorsehen von Löchern mit einem Durchmesser von weniger als 0,3 nm Wasser nicht durch die Stirnfläche dringen. Ab einem Durchmesser der Löcher bzw. Poren von 1 nm gelangt Wasser langsam durch die Stirnfläche 1 ins Innere der Hygienekappe. Bei einer Messdauer von 3 Sekunden können die Löcher bzw. Poren auch mit 3 - 4 nm gewählt werden, da das Wasser weniger rasch durch die Stirnfläche diffundiert als der Dampf. Wird eine Abdeckkappe 10 mit Löchern mit einer Dicke von etwa 3-4 nm mit der Stirnfläche 1 voran in Wasser getaucht, benötigt dieses je nach Dicke der Stirnfläche etwa 1 Minute bis es die Stirnfläche 1 durchdrungen hat.

Aufgrund der Materialeinheitlichkeit weisen die einzelnen Teile der Abdeckkappe 10 im Wesentlichen dieselben Eigenschaften auf. Einzig durch die jeweilige Dicke $d_S$ der Stirnwand 1, der Mantelwand 2 sowie der Grundwand 3 können spezielle Durchlässigkeiten für bestimmte Stoffe oder Strahlungen erzielt werden. So kann die Stirnwand 1 im Zuge des Herstellungsverfahrens besonders dünn ausgebildet werden, damit diese die Diffusion oder Strömung bestimmter Materialien, wie Gase, Flüssigkeiten, Atome, Moleküle, Verbindungen, zulässt.

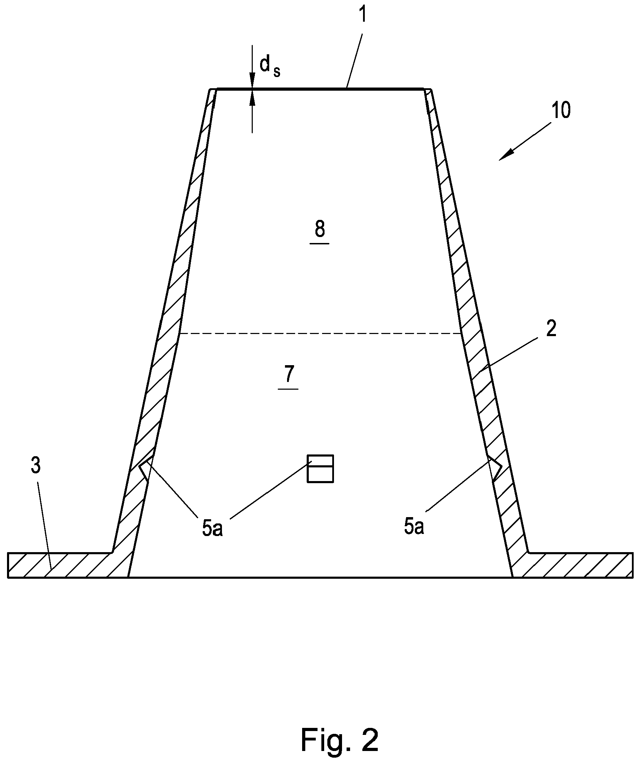

Das in **Fig. 2** dargestellte Ausführungsbeispiel entspricht im Wesentlichen dem in **Fig. 1a** und **Fig. 1b** dargestellten Ausführungsbeispiel, sodass lediglich auf die Unterschiede zwischen den beiden dargestellten Ausführungsbeispielen im Detail eingegangen wird. Die Höhe $h_1$ des an die Stirnwand 1 anschließenden Abschnittes 8 entspricht etwa der Höhe $h_2$ des der Stirnwand 1 fernen Verdickungsabschnittes 7.

Die Mantelwand 2 ist im vorliegenden Ausführungsbeispiel in zwei Abschnitte 7, 8 unterteilt, wobei beide Abschnitte 7, 8 umlaufend ausgebildet sind und die Abschnitte 7, 8 aneinander angrenzen und zwischen diesen beiden Abschnitten 7, 8 eine kreisförmige Begrenzungslinie verläuft, deren Punkte jeweils zur Stirnwand 1 denselben Normalabstand aufweisen. Die Dicke der Mantelwand 2 nimmt von der Stirnwand 1 aus, in axialer oder radialer Richtung gesehen, zu dem von der Stirnwand 1 fernen Ende der Mantelwand 2 hin kontinuierlich zu.

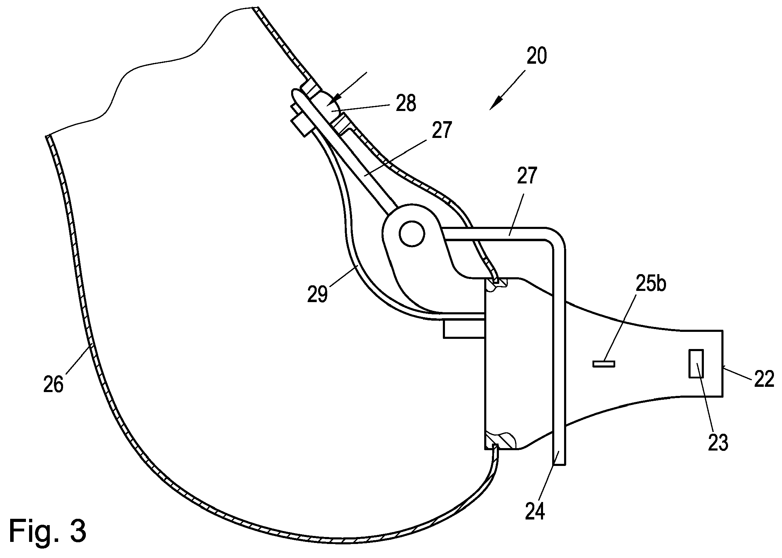

In **Fig. 3** ist eine Ausführungsform eines erfindungsgemäßen Messgerätes 20 näher dargestellt. Dieses Messgerät 20 weist ein Gehäuse 26 auf, das einen konisch zulaufenden Gehäuseteil 21 umfasst, der in weiterer Folge von einer vorstehend dargestellten Abdeckkappe 10 (**Fig. 4**) abgedeckt werden soll. Am Ende des konisch zulaufenden Gehäuseteils 21 ist eine Stirnfläche 22 angeordnet, an bzw. hinter der sich ein Sensor 23 befindet. Im vorliegenden Ausführungsbeispiel weist der konisch zulaufende Gehäuseteil 21 im Bereich der Stirnfläche 22 eine Öffnung auf, die zum Sensor 23 führt, sodass die zu messenden Gase und/oder Substanzen unmittelbar zum Sensor 23 strömen kann. Mit einer auf den konischen Gehäuseteil 21 aufgesetzten Abdeckkappe 10 (**Fig. 4**), kann gewährleistet werden, dass der Sensor 23 von Verschmutzungen frei gehalten wird.

Auf dem konisch zulaufenden Gehäuse 21 befindet sich eine Anzahl von Rastvorsprüngen 25b, alternativ oder zusätzlich kann auch eine Anzahl von Rastvorsprüngen 25a an der Mantelfläche des konisch zulaufenden Gehäuseteils 21 angeordnet sein. Wie in **Fig. 4** dargestellt, kann auf den konisch zulaufenden Gehäuseteil 21 die Abdeckkappe 10 aufgesetzt werden, sodass die vom konisch zulaufenden Gehäuseteil 21 abstehenden Rastvorsprünge 25b in die Rastausnehmungen 5a der Abdeckkappe 10 eingreifen. Um diesen abgedeckten Zustand des Messgeräts 20 zu erreichen, wird die Abdeckkappe 10, vorteilhafterweise mit zwei Fingern im unteren Verdickungsbereich 9 gegriffen und auf den konisch zulaufenden Gehäuseteil 21 aufgesetzt bzw. übergestülpt, sodass die Rastvorsprünge 25b des konisch zulaufenden Gehäuseteils 21 des Messgeräts in die Rastausnehmungen der Mantelwand 2 der Abdeckkappe 10 eingreifen. Die Abdeckkappe 10 ist dabei derart an den konisch zulaufenden Gehäuseteil 21 angepasst, dass sich die Rastvorsprünge bzw. Ausnehmungen 5a, 5b, 25a, 25b an aneinander angepassten Stellen befinden, sodass die Abdeckkappe 10 bzw. der konisch zulaufende Gehäuseteil 21 im verrasteten Zustand derart angeordnet sind, dass die Stirnfläche 1 über die Stirnwand 22 gestülpt ist und die an der Stirnfläche 22 des konisch zulaufenden Gehäuseteils 21 befindliche Ausnehmung vor dem Sensor 23 vollständig durch die Stirnwand 1 verdeckt oder abgedeckt wird. Dies wird insbesondere dadurch erreicht, dass die Abdeckkappe 10 in ihrem jeweiligen Abschnitt 8 mit geringer Dicke $d_2$ einer

besonders starken elastischen Verformung unterliegt und somit eine optimale Passform für das jeweilige Messgerät bzw. dessen konisch zulaufenden Gehäuseteil 21 ausbildet.

Die Lage und Position der Rastausnehmungen 25a und Rastvorsprünge 25b auf dem konisch zulaufenden Gehäuseteil 21 entspricht der Position der Rastausnehmungen 5a und/oder Rastvorsprünge 5b auf der Abdeckkappe 10a. Insbesondere sind die Rastausnehmungen 25a und die Rastvorsprünge 25b jeweils mit den selben Normalabstand zur Stirnfläche 22 auf dem konisch zulaufenden Gehäuseteil 21 angeordnet und in Umfangsrichtung vorteilhafterweise gleichmäßig verteilt. Die Gesamtzahl der Rastausnehmungen und/oder Rastvorsprünge 25a, 25b beträgt im vorliegenden Ausführungsbeispiel zwei, Altenativ können jedoch auch drei oder vier oder auch mehr Rastausnehmungen und/oder Rastvorsprünge vorgesehen sein. Die Form der Rastausnehmungen oder Rastvorsprünge 25a, 25b ist jeweils an die Form der Abdeckkappen 10 bzw. der Rastausnehmungen 5a bzw. Rastvorsprünge 5b der Abdeckkappe 10 angepasst. Die Rastvorsprünge 25a und stehen in einem Winkel von 10° bis 30°, höchstens 1 mm, insbesondere höchstens 0,5mm von der Außenwand des konisch zulaufenden Gehäuseteils 21 ab.

Das Messgerät 20 verfügt darüber hinaus über ein Auswurfelement 24, das in dem der vorderen Stirnwand 22 entfernten Ende des konisch zulaufenden Gehäuseteils angeordnet ist. Dieses Auswurfelement 24 ist von der vorderen Stirnwand 22 weiter entfernt als die Rastvorsprünge 25 des konisch zulaufenden Gehäuseteils. Das Auswurfelement 24 ist im vorliegenden Ausführungsbeispiel ringförmig, nämlich kreisringzylinderförmig, ausgebildet und umgibt den konisch zulaufenden Gehäuseteil 21. Wie aus **Fig. 4** ersichtlich, liegt die Grundwand 3 der Abdeckkappe 10 auf dem Auswurfelement 24 flächig auf. Eine Druckbeaufschlagung des Auswurfelements 24 zur Stirnwand 22 hin, bewirkt, dass die Abdeckkappe 10 aus ihrer Verrastung geschoben wird, wobei die Abdeckkappe im Bereich ihrer Rastausnehmung 5a während des Verschiebens aus der Verrastung einer elastischen Verformung unterliegt. Aufgrund des abgewinkelten Aufbaus der Rastausnehmung 5a sowie des Rastvorsprungs 25b wird beim Ausrasten die bei der elastischen Verformung der Abdeckkappe 10 aufgewendete Energie in Bewegungsenergie der Abdeckkappe 10 umgewandelt, welche die Abdeckkappe nach einer Richtung vom Auswurfelement 24 zur Stirnwand 21 hin beschleunigt.

Im vorliegenden Ausführungsbeispiel ist das Auswurfelement 24 einstückig mit einem Hebelfortsatz 27 ausgebildet. Der Hebelfortsatz 27 schließt an das Auswurfelement 24 an oder geht von diesem ab und ermöglicht eine Verschiebung oder Verschwenkung des Auswurfelements 24 zur Stirnwand 22 hin. Der Hebelfortsatz 27 ist in seinem Mittelbereich schwenkbar mit dem Gehäuse 26 verbunden, wobei der Hebelfortsatz 27 als zweiarmiger Hebel fungiert. An einem Ende dieses zweiarmigen Hebels ist das Auswurfelement 24 angeordnet, am anderen Ende des zweiarmigen Hebels ist ein Betätigungselement 28 in Form eines Druckknopfs angeordnet. Wird der Druckknopf gedrückt, so wird das dem Auswurfelement 24 ferne Ende des Hebelfortsatzes 27 in der Darstellung der **Fig. 4a** entgegen dem Uhrzeigersinn verschwenkt und das Auswurfelement 24 wird aufgrund der Schwenkbewegung zur vorderen Stirnwand 22 hin gedrückt. Aufgrund dieser Druckbeaufschlagung der Abdeckkappe 10 rasten die eingerasteten Rastausnehmungen bzw. Rastvorsprünge 25a, 5b aus ihrer eingerasteten Stellung aus und die Abdeckkappe 10 wird in Richtung der vorderen Stirnwand 22 weggeschleudert.

Durch die konkrete Anlenkung des Auswurfelements 24 aufgrund der Schwenkbewegung kann die Flugbahn der Abdeckkappe 10 besonders präzise eingestellt werden. Durch die besondere schwenkbare Ausbildung des Auswurfelements 24 werden die in diesem Ausführungsbeispiel einander gegenüberliegenden aneinander angepassten Kombinationen von Rastausnehmungen und Rastvorsprüngen zu unterschiedlichen Zeitpunkten gelöst. Hierdurch wird die Abdeckkappe 10 nicht gerade ausgeworfen sondern erfährt eine hiervon abweichende Flugbahn, die bei normaler Handhaltung des Geräts nach unten gerichtet ist und die Entsorgung gebrauchter Abdeckkappen erleichtert.

Eine weitere vorteilhafte Anordnung von Rastausnehmungen oder Rastvorsprüngen 25a, 5b kann, wie im Folgenden dargestellt, verwirklicht werden, indem ausschließlich zwei Rastausnehmungen oder Rastvorsprünge 25a, 5b vorgesehen sind, die denselben Abstand zu demjenigen Punkt aufweisen, an dem der Hebelfortsatz 27 an das Auswurfelements 24 anschließt oder von diesem abgeht. Hierdurch wird gewährleistet, dass die Verrastung der Abdeckkappe 10 mit dem Messgerät 20 für sämtliche miteinander verrasteten Rastausnehmungen und Rastvorsprünge 5b, 25a gleichzeitig ausrastet und keine Verlangsamung durch ein erneutes Einrasten eines anderen Rastvorsprungs in eine Rastausnehmung und erfolgt.

Somit kann eine möglichst große Abschussweite von Abdeckkappen mit dem jeweiligen Messgerät 20 erzielt werden. Dies hat den Vorteil, dass eine Entsorgung der Abdeckkappe über eine größere Distanz hinweg möglich ist und sich aufgrund der größeren Abschusskraft ein verbessertes Bediengefühl beim Entsorgen der Abdeckkappe einstellt und ganz allgemein die Motivation zur Benutzung sowie die Nutzerzufriedenheit gesteigert wird.

Um ein vorzeitiges oder unbeabsichtigtes Abschießen bzw. Entfernen einer Abdeckkappe aus dem verrasteten Zustand vom Messgerät 20 zu verhindern, ist im vorliegenden Ausführungsbeispiel eine Feder 29 in Form einer Drahtfeder vorgesehen, die den Hebelfortsatz 27 mit einer Vorspannung beaufschlagt, die das Auswurfelement 24 von der vorderen Stirnwand 22 wegdrückt. Erst wenn der Druck auf das Betätigungselement 28 die durch die Feder 29 erzeugte Vorspannung übersteigt wird der Abschuss der Abdeckkappe 10 veranlasst. Jedenfalls wird hierdurch vermieden, dass es zu einem unbeabsichtigten Abwerfen oder Auswurf der Abdeckkappe kommt.

Alternativ könnte diese Feder 29 auch an dem Auswurfelement 24 zugewandten Teil des Hebelfortsatzes 27 angreifen oder auch unmittelbar am Auswurfelement 24 selbst angreifen und dieses von der vorderen Stirnwand 22 wegziehen.

Im Folgenden wird ein Ausführungsbeispiel eines erfindungsgemäßen Verfahrens zur Herstellung einer Abdeckkappe 10 aus einer Kunststofffolie 31 dargestellt, wobei konkret die Herstellung des in den **Fig. 1a** und **Fig. 1b** dargestellten Ausführungsform einer erfindungsgemäßen Abdeckkappe erläutert wird.

**Fig. 5** zeigt eine Kunststofffolie 31 als Ausgangsmaterial, mit der die Abdeckkappe 10 ausgebildet werden soll. Die Kunststofffolie 31 weist im Ausgangszustand eine Dicke $d_f$ von 0,4 mm auf und besteht aus Polyethylenterephthalat Glycol (PETG).

Zur Ausformung einer Stirnfläche 1 mit einer Dicke $d_S$ von etwa 1 $\mu$m bis 10 $\mu$m wird ein Stempel 32 umfassend eine Anzahl von mehreren Teilstempeln 33 verwendet. Im vorliegenden Ausführungsbeispiel umfasst der Stempel 32 vier Teilstempel 33. Der Stempel 32 ist zylindrisch ausgebildet weist eine kreisförmige Stirnfläche mit einem Radius 1 mm auf. Jeder Teilstempel 33 weist eine kreissegmentförmige Stirnfläche in Form eines Viertelkreises mit einem Radius von 1 mm auf. Alle Teilstempel 33 sind separat zueinander verschiebbar und verschwenkbar angeordnet. In **Fig. 5a** ist eine Sicht auf die Teilstempel 33 von der Kunststofffolie 31 aus, wie durch die Schnittlinie **V a** angedeutet, dargestellt.

In einem ersten Ausformungsschritt liegen die Teilstempel 33 aneinander an, die viertelkreisförmigen Stirnflächen der Teilstempel 33 bilden gemeinsam eine kreisförmige Stirnfläche aus. Die Teilstempel 33 werden in dieser Stellung gemeinsam in den Bereich der Kunststofffolie 31 vorgeschoben. Wie in **Fig. 6** dargestellt, wird die Kunststofffolie 31 hierdurch verformt. Es wird ein Volumen 39 ausgebildet, das bis auf diejenige Seite, von der die Teilstempel 33 her in die Kunststofffolie 31 eingedrungen sind, von der Kunststofffolie 31 begrenzt ist. Die Außenform der aus der Kunststofffolie 31 ausgebildeten Form wird durch eine nicht dargestellte erste Gegenform festgelegt, die an der äußeren Mantelwand 2 sowie an der Stirnwand 1 der aus der Kunststofffolie 31 ausgebildeten Abdeckkappe 10 anliegt.

Im nächsten Schritt, dargestellt in **Fig. 7**, werden die Teilstempel 33 radial nach außen bewegt. Im vorliegenden Fall wird die Kunststofffolie 31 im Bereich der Teilstempel 33 gleichzeitig auf eine Temperatur von 40 °C erwärmt. Hierdurch wird die Kunststofffolie 31 im Zwischenbereich zwischen den Teilstempeln 33 stark gezerrt und verdünnt, sodass sich eine Stirnfläche 1 mit eine Dicke $d_S$ von etwa 4 $\mu$m ergibt.

Grundsätzlich ist vorgesehen, dass die Teilstempel 33 zunächst eine axiale Bewegung normal zur Ebene der Kunststofffolie 31 vollführen (**Fig. 6**) und erst wenn die Teilstempel 33 in dieser Richtung eine Endposition eingenommen haben, die Teilstempel 33 in radialer Richtung (**Fig. 7**) verschoben werden, um die Stirnfläche 1 auszubilden.

Alternativ ist es selbstverständlich möglich, die axiale Bewegung axiale Bewegung normal zur Ebene der Kunststofffolie 31 und die radiale Verschiebung der Teilstempel 33 nach außen miteinander zu kombinieren, sodass eine Überlagerung der beiden Bewegungen der Teilstempel 33 vorliegt.

Sofern eine rechteckige oder quadratische Stirnfläche 1 erstellt werden soll, ist es ausreichend, wenn die einzelnen Teilstempel 33 jeweils zu einem auszubildenden Eck der Stirnfläche 1 hin geführt werden, wie dies in **Fig. 7a** dargestellt ist. Sofern jedoch eine kreisrunde Stirnfläche 1 erstellt werden soll, können die Teilstempel 33 zusätzlich noch, wie in **Fig. 7b** dargestellt, in Rotationsbewegung um die gemeinsame Achse X versetzt werden. Die Rotationsbewegung wird von der radial nach außen gerichteten Bewegung der Teilstempel 33 überlagert. Die einzelnen Teilstempel 33 bewegen sich somit entlang eines spiralförmigen Bewegungspfads, wobei jeweils die kreiszylindrischen Mantelflächen der Teilstempel 33 jeweils mit der Kunststofffolie 31 in Kontakt stehen.

Wie bereits zuvor im Zusammenhang mit der Ausbildung von rechteckigen oder quadratischen Stirnflächen 1 beschrieben, ist auch eine kombinierte axiale und radiale Bewegung der Teilstempel 33 bei der Ausbildung von kreisförmigen Stirnflächen 1 möglich. In diesem Fall vollführen die Teilstempel 33 eine Bewegung entlang eines spiralförmigen Bewegungspfads, der schraubenförmig und sich nach unten hin erweiternd spiralförmig verläuft und eine Überlagerung aus einer axialen und einer radialen Bewegung sowie einer Rotationsbewegung um die Achse X darstellt.

Wie in **Fig. 7** dargestellt, weist ist ein Volumen 39 ausgebildet, das gegenüber einer gedachten Fortsetzung der Oberkante der Kunststofffolie 31 eine Höhe $h_1$ aufweist. Diese Höhe wird durch die Vorschublänge der einzelnen Teilstempel 33 axial in Richtung der Achse X festgelegt. Durch die Vorschubgeschwindigkeit der Teilstempel 33 sowie durch die Festlegung der Temperatur der Teilstempel 33 sowie der Kunststofffolie 31 in axialer Richtung wird die Dicke der das Volumen 39 seitlich begrenzenden Mantelwand 2 festgelegt, wobei insbesondere die Dicke in dem Bereich, in dem die Kunststofffolie 31 in die Mantelwand 2 übergeht besonders stark von der jeweiligen Vorschubgeschwindigkeit sowie der jeweiligen Temperatur abhängig ist.

Die Dicke der Mantelwand 2 kann durch die Auswahl der Temperatur der Kunststofffolie 31 sowie der Teilstempel 33 sowie durch die Vorschubgeschwindigkeit der Teilstempel 33 in axialer Richtung festgelegt werden. Die Dicke $d_1$ der Mantelwand 2 im ausgebildeten Mantelwandabschnitt 7 kann dabei kontinuierlich untersucht werden, wobei für den Fall, dass die Dicke $d_1$ zu gering ist, die Vorschubgeschwindigkeit der Teilstempel 33 in axialer Richtung verringert wird oder die Temperatur der Kunststofffolie 31 oder der Teilstempel 33 verringert wird. Ist die Dicke $d_1$ hingegen zu groß, wird die Vorschubgeschwindigkeit der Teilstempel 33 in axialer Richtung erhöht wird oder die Temperatur der Kunststofffolie 31 oder der Teilstempel 33 erhöht.

Im vorliegenden Ausführungsbeispiel wird eine Kunststofffolie 31 mit einer Dicke von 0,4 mm verwendet, die bei der Verarbeitung auf 40 °C erhitzt wurde. Die Teilstempel 33 haben eine Temperatur von 45°C und bilden ein Volumen 39 mit einer Höhe $h_1$ von 4 mm aus. Anschließend werden die Teilstempel 33, wie in **Fig. 7a** dargestellt, radial nach außen bewegt, wobei zunächst eine rechteckige Stirnfläche mit einer Seitenlänge von etwa 4 mm ausgebildet wird. In einem weiteren, in **Fig. 7b** dargestellten Schritt werden die Teilstempel 33 in eine Rotationsbewegung versetzt, wobei die Teilstempel um die Achse X rotieren. Die kreiszylindrischen Mantelflächen der Teilstempel 33 liegen dabei an der Innenwand der ausgebildeten Mantelwand an.

In **Fig. 8** und **Fig. 9** ist die Ausbildung weiterer Mantelwandabschnitte 8, 9 der Abdeckkappe 2 mit unterschiedlicher Dicke $d_2$, $d_3$ dargestellt. Anstelle der Teilstempel 33 wird in den folgenden Schritten ein weiterer Stempel 36 oder mehrere weitere Stempel verwendet, dessen Außenform der Form der jeweiligen Abdeckkappe 10 entspricht.

Wie in **Fig. 8** dargestellt, wird der weitere Stempel 36 in das ausgebildete Volumen 39 vorgeschoben, wobei die jeweilige Wanddicke $d_2$ des in diesem Verfahrensschritt ausgebildeten Bereichs der Mantelwand 2, im Folgenden Zwischenabschnitt 8 genannt, dünner ist als die Wanddicke $d_1$ des Bereichs der Mantelwand 2, im Folgenden erster Verdickungsabschnitt 7 genannt. Die Dicke $d_2$ der Mantelwand 2 im Zwischenabschnitt 8 wird, wie bereits bei der Erstellung des ersten Verdickungsabschnitts 7 ausgeführt, durch die Vorschubgeschwindigkeit des weiteren Stempels 36 sowie durch die Temperatur des weiteren Stempels 36 sowie die Temperatur der Kunststofffolie 31 bestimmt.

Die Außenform des aus der Kunststofffolie 31 ausgebildeten Teils der Abdeckkappe 10, insbesondere des Zwischenabschnitts 8, wird durch eine nicht dargestellte zweite Gegenform festgelegt, die an der äußeren Mantelwand 2 sowie an der Stirnwand 1 der aus der Kunststofffolie 31 ausgebildeten Abdeckkappe 10 anliegt.

Im vorliegenden Ausführungsbeispiel bleibt die Kunststofffolie 31 bei ihrer Verformung auf einer Temperatur von etwa 40°C bis 50°C, anschließend wird der weitere Stempel 36 in die zweite Gegenform eingebracht. Die zweite Gegenform ist auf eine Temperatur von etwa 40°C bis 50°C erhitzt. Der Zwischenbereich zwischen dem weiteren Stempel 36 und der zweiten Gegenform entspricht der gewünschten Form der Abdeckkappe 10 im ersten Verdickungsabschnitt 7 sowie im Zwischenabschnitt 8, im vorliegenden Ausführungsbeispiel ist die Wanddicke im Zwischenabschnitt 8 geringer als im ersten Verdickungsabschnitt 7.

Anschließend wird, wie in **Fig. 9** dargestellt, der weitere Stempel 36 weiter vorgeschoben, wobei ein zweiter Verdickungsabschnitt 9 ausgebildet wird, dessen Dicke $d_3$ größer ist als die Dicke $d_2$ im Zwischenabschnitt.

Die Außenform des aus der Kunststofffolie 31 ausgebildeten Abschnitts der Abdeckkappe 10, insbesondere des an den Zwischenabschnitt 8 anschließenden zweiten Verdickungsabschnitts 9, wird durch eine nicht dargestellte dritte Gegenform festgelegt, die an der äußeren Mantelwand 2 sowie an der Stirnwand 1 der aus der Kunststofffolie 31 ausgebildeten Abdeckkappe 10 anliegt.

Im vorliegenden Ausführungsbeispiel bleibt die Kunststofffolie 31 bei ihrer Verformung auf einer Temperatur von etwa 40°C bis 50°C, anschließend wird der weitere Stempel 36 in die zweite Gegenform eingebracht. Die dritte Gegenform ist auf eine Temperatur von etwa 40°C bis 50°C erhitzt. Der Zwischenbereich zwischen dem weiteren Stempel 36 und der dritten Gegenform entspricht der gewünschten Form der Abdeckkappe 10 im ersten Verdickungsabschnitt 7, im Zwischenabschnitt 8, sowie im zweiten Verdickungsabschnitt.

Fig. 10 zeigt die Fertigstellung der Abdeckkappe durch Abtrennen von der Kunststofffolie. Nach der Ausformung und Profilierung der gesamten Mantelwand 2 ist die Mantelwand noch mit der Kunststofffolie 31 verbunden. In einem abschließenden Schritt wird die Kunststofffolie 31 entlang einer vorgegebenen Schnittlinie 37 abgetrennt, im vorliegenden Ausführungsbeispiel wird die Kunststofffolie 31 entlang der Mantelwand gestanzt. Dadurch wird die Abdeckkappe 10 vom Rest der Kunststofffolie 31 abgetrennt.

Im vorliegenden Ausführungsbeispiel verläuft die Schnittlinie 37 kreisförmig und koaxial um die hohlkegelstumpfförmige Mantelfläche 2. Durch das Abschneiden entlang der Schnittlinie 37 wird eine Grundwand 3 der Abdeckkappe 10 ausgebildet, die parallel zur Stirnfläche radial von dem der Stirnfläche 1 fernen Ende der Mantelfläche 2 nach außen verläuft. Alternativ besteht auch die Möglichkeit, durch einen weiteren Verformungsprozess die Grundwand 3 von der Stirnwand 1 weg weisend und von der Mantelfläche 2 radial nach außen abstehend in einem Winkel von bis zu 20° insbesondere von bis zu 10° auszubilden.

Nach dem Ende der Ausformung, entweder vor oder nach dem Abschneiden der Abdeckkappe 10 entlang der Schnittlinie 37 wird die die Abdeckkappe 10 bildende Kunststofffolie 31 für einen vorgegebenen Zeitraum von 3 bis 4 Sekunden auf eine Temperatur von zwischen 50 und 120°C je nach verwendetem Kunststoff, im vorliegenden Fall auf 75 °C erhitzt. Im Zuge dieses Vorgangs wird der die Kunststofffolie 31 bildende Kunststoff durch die Erwärmung umgeformt. Die Kunststofffolie 31 verliert ihre thermoplastischen Eigenschaften. Durch die Wärmeeinwirkung werden die die Kunststofffolie 31 bildenden Molekülketten aufgespalten, wodurch eine größere Durchlässigkeit der Abdeckkappe 10, insbesondere im Bereich der Stirnfläche 1 geschaffen wird. Je länger die Wärmeeinwirkung auf die Kunststofffolie 31 dauert und je intensiver diese ist, insbesondere je höher die gewählte Temperatur ist, desto kürzer werden die die Kunststofffolie 31 bildenden Molekülketten und desto größer wird die Durchlässigkeit der Stirnfläche 1.

Alternativ können anstelle des Erhitzens der Kappe auch Löcher 6 in Form, im vorliegenden Fall Mikrolöcher oder Nanolöcher in der Stirnwand 1, vorzugsweise ausschließlich in der Stirnwand 1, ausgebildet werden, um eine erhöhte Durchlässigkeit für Moleküle einer bestimmten Größe zu erzielen. Die Ausbildung der Mikro- und Nanolöcher kann durch Verwendung eines Laserstrahls sowie durch Perforation durch Einstiche mit einer erhitzten Mikronadel erfolgen.

Besonders vorteilhaft ist es, wenn die Abdeckkappe 10 in dem der Umfangskante 4 fernen Bereich eine sehr hohe Steifigkeit aufweist, die der Stabilität der Abdeckkappe dient. Die Steifigkeit der Abdeckkappe 10 nimmt an der Mantelwand 2 zur Mitte hin zu, wobei die Mantelwand 2 in dem die Stirnwand 1 umgebenden Bereich wiederum eine größere Steifigkeit aufweist. Die Elastizität der Mantelwand 4 ist im Bereich ihrer dünnsten Stelle bzw. im Bereich ihrer Stelle mit der geringsten Steifigkeit am größten.

Vorzugsweise ist die Steifigkeit oder die Elastizität der Mantelwand 4 umfangsmäßig homogen verteilt und variiert lediglich mit dem Abstand zur Stirnwand 1.

## Patentansprüche

1. Abdeckkappe (10), insbesondere zum Aufsetzen auf ein Hautanalysegerät, umfassend eine von einer Umfangskante (4) begrenzte Stirnwand (1) sowie eine an der Umfangskante (4) der Stirnwand (1) anschließende Mantelwand (2), wobei zumindest ein Teil der Abdeckkappe (10) eine Dicke ($d_2$) aufweist, die geringer ist als diejenige Dicke ($d_3$) der Mantelwand (2) in einem Abschnitt (9) der Mantelwand (2), der im von der Stirnwand (1) abgewandten Bereich oder Ende der Mantelwand (2) liegt, **dadurch gekennzeichnet, dass** die Stirnwand (1) gasdurchlässig ausgebildet ist.

2. Abdeckkappe nach Anspruch 1, **dadurch gekennzeichnet, dass** die Mantelwand (2) zumindest einen umlaufenden Abschnitt (8) aufweist, dessen Dicke ($d_2$) geringer ist als diejenige Dicke ($d_3$) der Mantelwand (2) in einem Abschnitt (9) der Mantelwand (2), der im von der Stirnwand (1) abgewandten Bereich oder Ende der Mantelwand (2) liegt.

3. Abdeckkappe nach Anspruch 1 oder 2, **dadurch gekennzeichnet, dass** die Stirnwand (1) an zumindest einer Stelle eine Dicke ($d_S$) im Bereich von weniger als 100 $\mu$m, vorzugsweise zwischen 0,1 $\mu$m und 20 $\mu$m, insbesondere zwischen 5 $\mu$m und 10 $\mu$m, aufweist, wobei die Stirnwand (1) insbesondere eine über ihren Verlauf konstante Dicke (ds) aufweist.

4. Abdeckkappe (10) nach Anspruch 1, 2 oder 3, **gekennzeichnet durch** eine Grundwand (3), die die Mantelwand (2) an dem von der Stirnwand (1) abgewandten Ende, insbesondere einstückig, fortsetzt, und die vorzugsweise parallel oder in einem Winkel von höchstens 10° zur Stirnwand (1) von der Mantelwand (2) nach außen, insbesondere mit ansteigendem Normalabstand zur Stirnwand (1), verläuft, wobei die Grundwand (3) vorzugsweise kreisringförmig ausgebildet ist und insbesondere einen Außenradius zwischen 2 mm und 50 mm und/oder einen Innenradius zwischen 1 mm und 45 mm aufweist und/oder eine Kreisringbreite von 0,5 mm bis 35 mm aufweist.

5. Abdeckkappe (10) nach einem der vorangehenden Ansprüche, **dadurch gekennzeichnet, dass** die Mantelwand (2) im Bereich der Umfangskante (4) der Stirnwand (1) in einem Winkel ($\alpha$) von zwischen 20° und 95°, insbesondere zwischen 60° und 80°, zur Stirnwand (1) nach außen absteht, wobei sich vorzugsweise die Mantelwand (2), insbesondere konisch, erweitert.

6. Abdeckkappe (10) nach einem der vorangehenden Ansprüche, **dadurch gekennzeichnet,**

   - **dass** die Abdeckkappe (10) in dem der Stirnwand (1) abgewandten Ende der Mantelwand (2) Rastausnehmungen (5a) und/oder Rastvorsprünge (5b) zur lösbaren Befestigung an einer Messvorrichtung vorgesehen sind, und/oder
   - **dass** insbesondere die einzelnen Rastausnehmungen (5a) und/oder Rastvorsprünge (5b) jeweils denselben Normalabstand zur Stirnwand (1) aufweisen und/oder in Umfangsrichtung der Mantelwand (2) gleichmäßig verteilt sind, und/oder wobei die Gesamtzahl der Rastausnehmungen (5a) und/oder Rastvorsprünge (5b) zwei oder vier beträgt, und/oder
   - **dass** insbesondere die Rastausnehmungen (5a) und/oder Rastvorsprünge (5b) in einem Winkel von ($\beta_1$, $\beta_2$) 5° bis 40° und/oder höchstens 2 mm, insbesondere höchstens 0,3 mm von der Mantelwand (2) abstehen oder in diese eindringen.

7. Abdeckkappe (10) nach einem der vorangehenden Ansprüche, **dadurch gekennzeichnet, dass** die Stirnwand (1) strahlungs-, dampf-, feuchtigkeits-, partikel-, und/oder lichtdurchlässig ausgebildet ist und/oder eine Anzahl von, insbesondere dampfdurchlässigen oder gasdurchlässigen, Mikrolöchern (6) aufweist.

8. Abdeckkappe (10) nach einem der vorangehenden Ansprüche, **dadurch gekennzeichnet, dass** die Abdeckkappe (10) materialeinheitlich und/oder einstückig ausgebildet ist, und insbesondere aus thermoplastischem oder plastisch verformten oder verformbaren Kunststoff, vorzugsweise aus einem der folgenden Materialien oder einer Mischung hiervon, besteht:

- PETG (Polyethylenterephthalat Glycol),
- PP (Polypropylen),
- PE (Polyethylen),
- PC (Polycarbonat),
- PVC (Polyvinylchlorid),
- PS (Polystyrol),
- ABS (Acrylonitrile butadiene styrene),
- HDPE (High Density Polyethylene),
- LDPE (Low Density Polyethylene),
- PET (Polyethylene terephthalate),
- PMMA (Polymethyl methacrylate),
- ECOTERM S 900 T1,
- PETG / Copolyester 67639,

wobei das Material der Abdeckkappe (10), insbesondere zusätzlich, einen oder mehrere der folgenden Bestandteile enthält:

- Additive,
- Stabilisatoren,
- Farbmittel,
- Füllstoffe,
- Verstärkungsstoffe,

wobei insbesondere das Material der Abdeckkappe (10) einen Farbstoff aufweist und die Stirnwand (1) für bestimmte, insbesondere sichtbare, Wellenlängenanteile undurchsichtig oder absorbierend ausgebildet ist.

9. Abdeckkappe (10) nach einem der vorangehenden Ansprüche, **dadurch gekennzeichnet, dass** die Dicke ($d_2$) der Mantelwand (2) von der Stirnwand (1) zu dem von der Stirnwand (1) fernen Ende der Mantelwand (2) hin, insbesondere zumindest über einen Teilbereich der Mantelwand (2), vorzugsweise kontinuierlich, zunimmt.

10. Abdeckkappe (10) nach einem der vorangehenden Ansprüche, **dadurch gekennzeichnet, dass** die Mantelwand (2) im Nahebereich der Stirnwand (1) einen, insbesondere an die Umfangskante (4) anschließenden, ersten Verdickungsabschnitt (7) aufweist, in dem die Dicke ($d_1$) der Mantelwand (2) die Dicke ($d_S$) der Stirnwand (1) übersteigt, dass die Mantelwand (2) einen an den ersten Verdickungsabschnitt (7) anschließenden Zwischenabschnitt (8) aufweist, wobei die Dicke ($d_2$) der Mantelwand (2) im Zwischenabschnitt (8) geringer ist als die Dicke ($d_1$) der Mantelwand (2) im ersten Verdickungsabschnitt (7), dass die Mantelwand (2) einen an den Zwischenabschnitt (8) anschließenden und bis zu dem der Stirnwand (1) fernen Ende der Mantelwand (2) reichenden zweiten Verdickungsabschnitt (9) aufweist, und dass die Dicke ($d_3$) der Mantelwand (2) im zweiten Verdickungsabschnitt (9) größer ist als die Dicke ($d_2$) der Mantelwand (2) im Zwischenabschnitt (8).

11. Abdeckkappe (10) nach einem der vorangehenden Ansprüche, **dadurch gekennzeichnet, dass** die Dicke der Mantelwand (2), insbesondere in axialer oder radialer Richtung, einen über die Mantelwand (2) kontinuierlichen, knick- und kantenfreien Verlauf aufweist, und/oder als Dicke ($d_1$, $d_2$, $d_3$) die jeweils mittlere Dicke im jeweiligen Abschnitt (7, 8, 9) der Mantelwand (2) gilt, und/oder dass die Mantelwand (2), insbesondere mit Ausnahme des Bereichs der Rastvorsprünge (5a) und/oder Rastausnehmungen (5b), rotationssymmetrisch ausgebildet ist.

12. Abdeckkappe (10) nach Anspruch 8 oder 9, **dadurch gekennzeichnet, dass** die Dicken ($d_1$, $d_2$, $d_3$) des ersten Verdickungsabschnitts (7), des Zwischenabschnitts (8) und des zweiten Verdickungsabschnitts (9) zueinander im folgenden Verhältnis stehen:

$$2 < d_1 : d_2 < 5 \text{ und/oder } 2 < d_3 : d_2 < 5 \text{ und/oder } 0,8 < d_1 : d_3 < 1,25$$

und/oder dass die Dicken ($d_1$, $d_2$, $d_3$) des ersten Verdickungsabschnitts (7), des Zwischenabschnitts (8) und des zweiten Verdickungsabschnitts (9) wie folgt festgelegt sind:

$$150 \text{ µm} < d_1 < 250 \text{ µm und/oder } 50 \text{ µm} < d_2 < 100 \text{ µm und/oder } 150 \text{ µm} < d_3 < 250 \text{ µm.}$$

**13.** Abdeckkappe (10) nach einem der Ansprüche 8 bis 10, **dadurch gekennzeichnet, dass** die Höhe ($h_1$) des ersten Verdickungsabschnitts (7), die Höhe ($h_2$) des Zwischenabschnitts (8) sowie die Höhe ($h_3$) des zweiten Verdickungs- abschnitts (9) in folgendem Verhältnis stehen:

$$0,2 < h_1 : h_2 < 0,6 \text{ und/oder } 0,8 < h_3 : h_2 < 1,25 \text{ und/oder } 0,2 < h_1 : h_3 < 0,6.$$

und/oder

- dass die Mantelwand (2) eine Höhe von 2 mm bis 80 mm aufweist und/oder
- dass das Verhältnis zwischen der Höhe und der maximalen Abmessung, insbesondere der Durchmesser oder die Diagonale, der Stirnwand (1) zwischen 0,01 und 55 liegt.

**14.** Abdeckkappe (10) nach einem der vorangehenden Ansprüche, **dadurch gekennzeichnet, dass** die Stirnwand (1) kreisförmig ausgebildet ist und insbesondere einen Durchmesser von 1 mm bis 50 mm aufweist, oder dass die Stirnwand (1) quadratisch oder rechteckig ausgebildet ist und wobei die Seitenkanten der Stirnwand (1) eine Länge von zwischen 2 mm und 40 mm aufweisen.

Fig. 1a

Fig. 1b

EP 3 056 146 A1

Fig. 2

Fig. 3

Fig. 4

Fig. 4a

Fig. 5a

Fig. 5

Fig. 6

Fig. 7b

Fig. 7

Fig. 7a

EP 3 056 146 A1

Fig. 8

Fig. 9

Fig. 10

Europäisches
Patentamt

European
Patent Office

Office européen
des brevets

## EUROPÄISCHER RECHERCHENBERICHT

**Nummer der Anmeldung**

EP 16 16 1365

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/329951 A1 (JUNG SUNG-KWON [US] ET AL) 30. Dezember 2010 (2010-12-30) * Absätze [0035] - [0038], [0045] - [0053], [0060] - [0066]; Ansprüche; Abbildungen * ----- | 1-14 | INV. A61B5/145 A61B5/1455 A61B5/00 G01J5/02 B29C51/08 |
| X | US 2001/034479 A1 (RING LAWRENCE S [US] ET AL) 25. Oktober 2001 (2001-10-25) * Absätze [0035] - [0041], [0063]; Ansprüche; Abbildungen * ----- | 1-7,9,14 | ADD. G01K1/08 |

**RECHERCHIERTE SACHGEBIETE (IPC)**

A61B
G01J
B65D
B21D
G01K
B29C
C08J
B01D

Der vorliegende Recherchenbericht wurde für alle Patentansprüche erstellt

| Recherchenort | Abschlußdatum der Recherche | Prüfer |
|---|---|---|
| Den Haag | 28. April 2016 | Crisan, Carmen-Clara |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.......................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 16 16 1365

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-04-2016

| Im Recherchenbericht angeführtes Patentdokument | Datum der Veröffentlichung | Mitglied(er) der Patentfamilie | Datum der Veröffentlichung |
|---|---|---|---|
| US 2010329951 A1 | 30-12-2010 | AT 488173 T | 15-12-2010 |
| | | EP 1845839 A2 | 24-10-2007 |
| | | EP 2281502 A1 | 09-02-2011 |
| | | US 2008159913 A1 | 03-07-2008 |
| | | US 2010329951 A1 | 30-12-2010 |
| | | US 2013343963 A1 | 26-12-2013 |
| | | US 2014193894 A1 | 10-07-2014 |
| | | WO 2006083892 A2 | 10-08-2006 |
| US 2001034479 A1 | 25-10-2001 | AU 5133901 A | 12-11-2001 |
| | | US 2001034479 A1 | 25-10-2001 |
| | | WO 0182792 A1 | 08-11-2001 |

Für nähere Einzelheiten zu diesem Anhang : siehe Amtsblatt des Europäischen Patentamts, Nr.12/82

EPO FORM P0461